(19) Europäisches Patentamt
European Patent Office
Office européen des brevets

(11) EP 1 802 297 B1

(12) **EUROPEAN PATENT SPECIFICATION**

(45) Date of publication and mention of the grant of the patent:
**30.03.2011 Bulletin 2011/13**

(21) Application number: **05812101.3**

(22) Date of filing: **19.10.2005**

(51) Int Cl.:
***A61K 31/33*** *(2006.01)* ***A61K 31/341*** *(2006.01)*
***A61K 31/41*** *(2006.01)* ***A61K 31/4196*** *(2006.01)*
***A61K 31/427*** *(2006.01)* ***A61K 31/429*** *(2006.01)*
***A61K 31/515*** *(2006.01)* ***A61P 31/06*** *(2006.01)*
***C07D 249/08*** *(2006.01)* ***C07D 257/04*** *(2006.01)*

(86) International application number:
**PCT/US2005/037537**

(87) International publication number:
**WO 2006/047162 (04.05.2006 Gazette 2006/18)**

(54) **COMPOSITIONS AND METHODS FOR TREATMENT OF DISEASE CAUSED BY YERSINIA SPP INFECTION**

ZUSAMMENSETZUNGEN UND VERFAHREN ZUR BEHANDLUNG VON KRANKHEITEN, DIE DURCH INFEKTION MIT YERSINIA SPP AUSGELÖST SIND

COMPOSITIONS ET PROCEDES DE TRAITEMENT DE MALADIES CAUSEES PAR L'INFECTION YERSINIA SPP

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.10.2004 US 621442 P**

(43) Date of publication of application:
**04.07.2007 Bulletin 2007/27**

(73) Proprietor: **The Burnham Institute**
**La Jolla, CA 92037 (US)**

(72) Inventors:
- **MUSTELIN, Tomas, Michael**
  **San Diego, CA 92129 (US)**
- **PELLECCHIA, Maurizio**
  **San Diego, CA 92103 (US)**
- **TAUTZ, Lutz**
  **La Jolla, CA 92037 (US)**

(74) Representative: **Goodfellow, Hugh Robin et al**
**Carpmaels & Ransford**
**One Southampton Row**
**London**
**WC1B 5HA (GB)**

(56) References cited:

| | |
|---|---|
| **WO-A-03/073999** | **WO-A-03/074497** |
| **WO-A-03/097621** | **WO-A-2005/007141** |
| **WO-A-2005/076695** | **FR-A- 2 858 324** |
| **GB-A- 2 376 944** | **GB-A- 2 386 892** |
| **GB-A- 2 387 172** | **US-A- 4 185 021** |
| **US-A1- 2002 052 396** | **US-A1- 2002 091 148** |
| **US-A1- 2003 229 065** | **US-A1- 2003 229 065** |
| **US-A1- 2004 002 526** | **US-A1- 2004 138 104** |
| **US-A1- 2004 171 629** | **US-A9- 2005 042 674** |
| **US-B1- 6 440 985** | |

- **DOMAN T N ET AL: "Molecular Docking and High-Throughput Screening for Novel Inhibitors of Protein Tyrosine Phosphatase-1B" JOURNAL OF MEDICINAL CHEMISTRY, AMERICAN CHEMICAL SOCIETY. WASHINGTON, US, vol. 45, no. 11, 2002, pages 2213-2221, XP003003278 ISSN: 0022-2623**
- **ZERVOSEN A ET AL: "INTERACTIONS BETWEEN PENICILLIN-BINDING PROTEINS (PBPs) AND TWO NOVEL" ANTIMICROBIAL AGENTS AND CHEMOTHERAPY, AMERICAN SOCIETY FOR MICROBIOLOGY, WASHINGTON, DC, US, vol. 48, no. 3, March 2004 (2004-03), pages 961-969, XP003005798 ISSN: 0066-4804**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 802 297 B1

- HUANG NIU ET AL: "Identification of non-phosphate-containing small molecular weight inhibitors of the tyrosine kinase p56 Lck SH2 domain via in silico screening against the pY + 3 binding site" JOURNAL OF MEDICINAL CHEMISTRY, vol. 47, no. 14, 1 July 2004 (2004-07-01), pages 3502-3511, XP002469918 ISSN: 0022-2623
- RODRIGUES CARLOS R ET AL: "CoMFA and HQSAR of acylhydrazide cruzain inhibitors" BIOORGANIC AND MEDICINAL CHEMISTRY LETTERS, vol. 12, no. 11, 3 June 2002 (2002-06-03), pages 1537-1541, XP002469919 ISSN: 0960-894X
- HOLLA B SHIVARARNA ET AL: "Synthesis and antibacterial studies of a new series of 1,2-bis (1,3,4-oxadiazol-2-yl)ethanes and 1,2-bis(4-amino-1,2,4-triazo l-3-yl)ethanes" EUROPEAN JOURNAL OF MEDICINAL CHEMISTRY, vol. 35, no. 2, February 2000 (2000-02), pages 267-271, XP002469920 ISSN: 0223-5234
- DATABASE REGISTRY Chemical Library, Supplier: Interbioscrenn Ltd. 19 July 2001 (2001-07-19), XP002468278 retrieved from STN accession no. 346713-48-2
- DATABASE REGISTRY CHEMICAL ABSTRACTS SERVICE, COLUMBUS, OHIO, US; Chemical Library; Supplier: Interbioscreen Ltd. 17 September 2001 (2001-09-17), XP002468279 retrieved from ST accession no. 357176-11-5
- DATABASE REGISTRY Chemical Library; Suppl.: Chembridge Corporation 25 April 2004 (2004-04-25), XP002468281 retrieved from STN accession no. 676590-13-9
- DATABASE REGISTRY Chemical Library; Supplier: Labotest 10 January 2002 (2002-01-10), XP002468280 retrieved from STN accession no. 381671-34-7
- DATABASE CA [Online] CHEMICAL ABSTRACTS SERVICE, COLUMBUS, OHIO, US; HILLERS, S. ET AL: "Tuberculostatic action of some 5(-nitrophenyl) furfural derivatives" XP002469922 retrieved from STN Database accession no. 1969: 500102
- BLASKOVICH M A ET AL: "Recent discovery and development of protein tyrosine phosphatase inhibitors" EXPERT OPINION ON THERAPEUTIC PATENTS 2002 UNITED KINGDOM, vol. 12, no. 6, 2002, pages 871-905, XP002469921 ISSN: 1354-3776
- TAUTZ LUTZ ET AL: "Inhibition of Yersinia tyrosine phosphatase by furanyl salicylate compounds" JOURNAL OF BIOLOGICAL CHEMISTRY, AMERICAN SOCIETY OF BIOLOCHEMICAL BIOLOGISTS, BIRMINGHAM,, US, vol. 280, no. 10, March 2005 (2005-03), pages 9400-9408, XP002434665 ISSN: 0021-9258
- CHEN YEN TING ET AL: "Parallel synthesis of a library of bidentate protein tyrosine phosphatase inhibitors based on the alpha-ketoacid motif" BIOORGANIC & MEDICINAL CHEMISTRY, vol. 12, no. 12, 15 June 2004 (2004-06-15), pages 3289-3298, XP002469937 ISSN: 0968-0896
- TAUTZ L.: 'Inhibition of Yersinia Tyrosine Phosphatase by Furanyl Salicylate Compounds' THE JOURNAL OF BIOLOGICAL CHEMISTRY vol. 280, March 2005, pages 9400 - 9408, XP002434665
- TITBALL R.W.: 'Vacciation Against Bubonic and Pneumonic Plaque' VACCINE vol. 19, 2001, pages 4175 - 4184, XP004255134

## Description

### FIELD

The invention generally relates to compositions and methods for treatment of disease caused by *Yersinia* spp. infection. More specifically, the invention relates to protein tyrosine phosphatase inhibitors and salt froms thereof and in particular those for use in treating a disease state in a manual caused by Yersinia spp. infection.

### BACKGROUND

To survive in humans, pathogenic bacteria have evolved numerous mechanisms to evade the host's immune response. Ernst, Cell. Microbiol. 2: 379-386, 2000; DeVinney, et al., Trends Microbiol. 8: 29-33, 2000. One of the most successful strategies was adopted by *Yersinia pestis,* namely a type III secretion system that injects a set of paralyzing proteins directly into the cytoplasm of macrophages and lymphocytes that the bacterium encounters in the lymph nodes of infected individuals. Persson, et al., Mol. Microbiol. 18: 135-150, 1995; Cheng, et al., J. Bacteriol. 182: 3183-3190, 2000. As a result, the targeted cells become unable to respond, and the bacteria can multiply unopposed by the normal mechanisms of host defense.

The natural route of *Yersinia pestis* infection is by transmission from infected rats or other animals by blood-sucking fleas, which are weakened by the bacteria in their gut and therefore expel bacterial mass into the epidermis of their next victim when trying to feed. From these flea bites, the bacteria travel to local lymph nodes, where they multiply and cause a massive lymphadenitis within 2-6 days. Autenrieth, et al. Infect. Immun. 61: 2585-2595, 1993; Autenrieth, et al., Immunobiology 187: 1-16, 1993; Autenrieth, et al., J. Med. Microbiol. 44: 285-294, 1996. These enlarged and painful lymph nodes, or 'bubos', give the disease its common name Bubonic Plague. Unless treated with high-dose streptomycin or tetracycline type antibiotics during the first few days, the infection develops into a toxemic sepsis, which is often fatal. Titball, et al., British Medical Bulletin 54: 625-633, 1998; Hinnebusch, J. Mol. Med. 75: 645-652, 1997. A normally vary rare, but much more rapidly lethal, form of the infection is caused by inhaled bacteria and is referred to as pneumonic plague or plague pneumonia. By this route of infection, the number of bacteria entering the body can be much larger than from microscopic flea bites and the bacteria are efficiently disseminated to the peritracheal, mediastinal, and other central lymph nodes, from which they gain access to the blood stream much earlier. Although several vaccines exist and *Yersinia* usually is sensitive to antibiotics, the pneumonic form of the disease is difficult to diagnose and still often results in death. Cleri, et al., Semin. Resp. Infect. 12: 12-23, 1997; Titball, et al., Vaccine 19: 4175-4184, 2001; Friedlander, et al., Clin. Infect. Dis. 21: S178-181, 1995.

Despite efforts to eradicate the disease, natural reservoirs of *Yersinia pestis* still exist in wild rats and other rodent populations in parts of Africa, southeast Asia, and southwestern United States, and sporadic human cases of plague still occur every year. Christie, Ecol. Dis. 1:111-115, 1982. Although these cases pale by comparison to the devastating pandemics that killed an estimated 200 million people, mostly in Europe, during historical times, the World Health Organization now recognizes plague as a re-emerging public health concern. Titball, et al., British Medical Bulletin 54: 625-633, 1998; Hinnebusch, J. Mol. Med. 75: 645-652, 1997. There are also increasing fears that *Yersinia pestis* may be used for biological warfare or bioterrorism. McGovern, et al., Arch. Dermatol. 135: 311-322, 1999; Inglesby, et al., JAMA 283: 2281-2290, 2000; Hawley, et al., Annu. Rev. Microbiol. 55: 235-253, 2001. The potential threat is heightened by the existence of multidrug-resistant strains of *Yersinia pestis* and the rapidly lethal course of the pneumonic form of the disease caused by aerosolized *Yersinia.* Galimand, et al., N. Engl. J. Med. 337: 677-680, 1997; Guiyoule, et al., Emerg. Infect. Dis. 7: 43-48, 2001. New approaches to combat plague are urgently needed.

The molecular mechanisms employed by all virulent strains of *Yersinia pestis* and two related species, *Y. pseudotuberculosis* and *Y. enterocolitica,* are based on an extrachromosomal virulence plasmid, which encodes a type III secretion system and several effector proteins called Yops (*Yersinia* Outer membrane Proteins). Cornelis, et al., Mol. Microbiol. 23: 861-867, 1997; Cornelis, et al., Microbiol. Mol. Biol. Rev. 62: 1315-1352, 1998. The type III secretion system is a highly conserved macromolecular machinery found in many pathogenic Gram-negative bacteria, and is induced by contact with a eukaryotic cell to inject effector Yops into the cytoplasm of the target cells. Juris, et al., Cellular Microbiology 4: 201-211, 2002. In the host cell, the Yops disrupt signaling cascades responsible for initiating key immune functions, such as phagocytosis, respiratory burst, cytokine production, and lymphocyte activation. Black, et al., EMBO J. 16: 2730-2744, 1997; Persson, et al., EMBO J. 16: 2307-2318, 1997; Andersson, et al., Mol. Microbiol. 20: 1057-1069, 1996; Aepfelbacher, et al., Biol. Chem. 380: 795-802, 1999; Green, et al., J. Leuk Biol. 57: 972-977, 1995; Yao, et al., J. Exp. Med. 190: 1343-1350, 1999. As a consequence, both the innate and adaptive immune responses are seriously impaired. Cornelis, Proc. Natl. Acad. Sci. USA 97: 8778-8783, 2000. However, a protective immunity can be acquired by vaccination. Titball, et al., Vaccine 19: 4175-4184, 2001; Friedlander, et al., Clin. Infect. Dis. 21: S178-181, 1995.

YopH is a 468-amino acid, exceptionally active, protein tyrosine phosphatase (PTPase) with a C-terminal catalytic domain and a multifunctional N-terminal domain, which binds tyrosine-phosphorylated target proteins. Guan,

et al., Science 249: 553-556, 1990; Bliska, et al., Proc. Natl. Acad. Sci USA 88: 1187-1191, 1991; Montagna, et al., J. Biol. Chem. 276: 5005-5011, 2001; Evdokimov, et al., Acta Cryst. 57: 793-799, 2001. The catalytic domain of YopH is structurally very similar to that of eukaryotic PTPases. Stuckey, et al., Nature 370: 571-575, 1994. A marked dephosphorylation of proteins in human epithelial cells and murine macrophages has been observed during infection with live bacteria. Andersson, et al., Mol. Microbiol. 20: 1057-1069, 1996; Bliska, et al., Proc. Natl. Acad Sci USA 88: 1187-1191, 1991; Bliska, et al., J. Exp. Med. 176: 1625-1630, 1992; Hartland, et al., Infect. Immun. 62: 4445-4453, 1994. In macrophages and neutrophils; the dephosphorylated proteins include the focal adhesion proteins Cas, focal adhesion kinase, and paxillin, providing a molecular mechanism for inhibition of migration and phagocytosis by these cells. Persson, et al., EMBO J. 16: 2307-2318, 1997; Black, et al., EMBO J. 16: 2730-2744, 1997; Black, et al., Mol. Microbiol. 29: 1263-1274, 1998; Ruckdeschet, et al., Inject. Immun. 84: 724-733, 1996. A YopH inhibitor, aurintricarboxylic acid, has been shown to block *in vitro* and *in vivo* activity of YopH virulence factor of *Yersinia pestis.* Liang, et al. J. Biol. Chem. 278: 41734-41741, 2003.

WO 03/097621A describes "methods of using thiazolidinedithione derivatives to treat cancer, neurodegenerative disease, diabetes, renal disease or inflammation in a mammal and pharmaceutical compositions containing such derivatives are disclosed". Compounds of the following form are described.

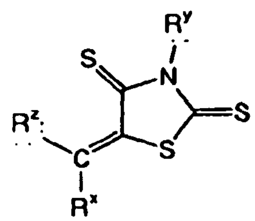

wherein:

$R^x$ is heterocyclyl;
$R^y$ is hydrogen, alkyl, alkenyl, aryl, aralkyl, aralkenyl, cycloalkyl, cycloalkylalkyl, haloalkyl, haloalkenyl, heterocyclylalkyl, $-R^a-O-R^b$, $-R^a-N(R^b)_2$, $-R^a-C(O)OR^b$, $-R^a-C(O)N(R^b)_2$, $-R^a-N=N-OR^c$, or $-R^a-N(R^b)C(O)OR^c$;
$R^a$ is hydrogen, alkyl, aralkyl, aryl, haloalkyl, cycloalkyl, cycloalkylalkyl, heterocyclyl or heterocyclylalkyl;
each $R^a$ is independently an optionally substituted straight or branched alkylene or alkenylene chain;
each $R^b$ is independently hydrogen, alkyl, alkenyl, cycloalkyl, cycloalkylalkyl, aralkyl or aryl; and
each $R^c$ is independently hydrogen, alkyl or aralkyl;

Doman et al. J. Med. Chem. 45: 2213-2221, 2002 describes the use of "high-throughput screening (HTS) of compound libraries... to discover... novel inhibitors for the enzyme protein tyrosine phosphatase-1B (PTP1B), a tyrosine phosphatase that has been implicated as a key target for type II diabetes".
Chen Yen Ting et al. Bio. Med. Chem. 12: 3289-3298, 2004 "describes the synthesis, using parallel solution-phase methods, of a library of 104 potential inhibitors of PTPases".

A need exists in the art for new treatments for *Yersinia* spp. infection. New approaches to combat plague caused by *Yersinia* spp., for example, *Y. pestis, Y. pseudotuberculosis,* or *Y. enterocolitica,* are urgently needed. The present invention is directed to these, as well as other important ends.

## SUMMARY

The invention is generally related to inhibitors as set out in claims 1 and 2.

A method is also disclosed for treating a disease state in a mammal caused by *Yersinia* spp. infection comprises the step of administering to the mammal a therapeutic amount of a compound of formula:

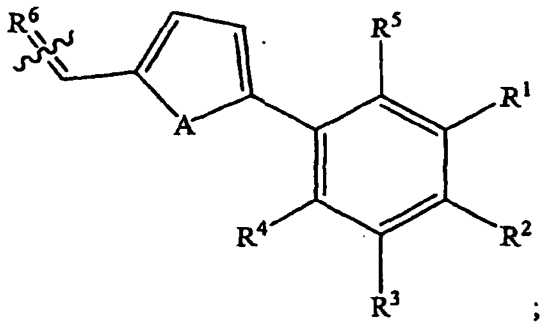

wherein:

A is O, NH or S;
$R^1$, $R^2$, and $R^3$ are each independently H, -OH, $-OR^7$, $NO_2$, or -(C=O)OH, provided that at least one of $R^1$, $R^2$, and $R^3$ is -OH, $-NO_2$, or -(C=O)OH;
$R^4$ and $R^5$ are each independently H, -OH, $C_1$-$C_6$ alkyl or halo;
$R^6$ is:

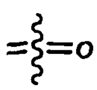 , 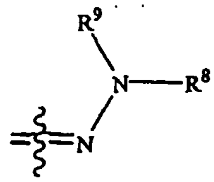 , 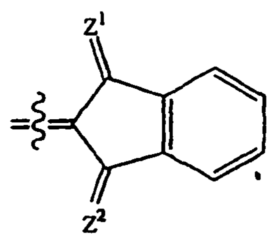 , 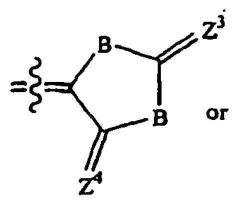 or 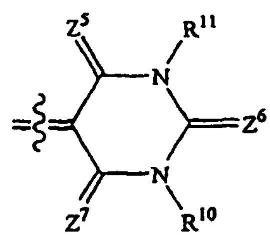

$R^7$ is H, alkyl or aralkyl;
$R^8$ and $R^9$ are each independently H, alkyl, aralkyl, alkanoyl, aralkanoyl or heteroaralkanoyl, or $R^8$ and $R^9$ taken together with the nitrogen atom to which they are attached form a five to eight membered heteroaryl ring having from one to four O, N or S heteroatoms, wherein the heteroaryl ring is optionally substituted;
$R^{10}$ and $R^{11}$ are each independently H or $C_1$-$C_6$ alkyl;
B is $NR^{12}$ or S;
$Z^1$ and $Z^2$ are O;
$Z^3$ and $Z^4$ are each independently O, S or $NR^{13}$;
$Z^5$, $Z^6$ and $Z^7$ are each independently S or O; and
$R^{12}$ and $R^{13}$ are each independently H, alkyl, aralkyl, N-aralkylcarbamoylalkyl or aralkyl-N(H)-C(=O)-alkyl, or $R^{12}$ and $R^{13}$ taken together with the atoms through which they are connected form an imidazole or benzimidazole ring, optionally substituted. In a preferred embodiment, $R^{12}$ and $R^{13}$ are each independently H, alkyl, aralkyl, N-aralkylcarbamoylmethyl or aralkyl-N(H)-C(=O)-methyl.

In the method disclosed the disease state is caused by *Yersinia* spp. infection. The *Yersinia* spp. is selected from *Yersinia pestis, Yersinia pseudotuberculosis,* or *Yersinia enterocolitica.* In the method disclosed the disease state is responsive to treatment with a *Yersinia* spp. protein tyrosine phosphatase inhibitor.
In further disclosures $R^6$ is

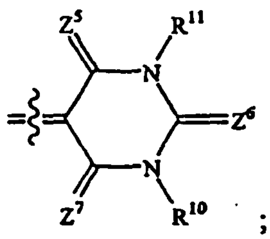

$R^1$ is -OH; $R^2$ is -(C=O)H; $R^3$ is H; $R^4$ is H; and $R^5$ is H; A is O; $R^{10}$ is H; $R^{11}$ is H; $Z^5$ is O; $Z^6$ is S; and $Z^7$ is O. In a detailed aspect, the compound is 4-[5-(4,6-dioxo-2-thioxo-tetrahydro-pyrimidin-5-ylidenemethyl)-furan-2-yl]-2-hydroxy-benzoic acid.

The method disclosed further provides a compound wherein,
$R^6$ is

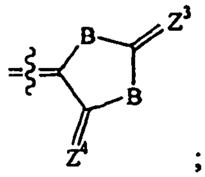

B is S or $NR^{12}$; $Z^3$ is $NR^{13}$; $Z^4$ is O; and $R^{12}$ and $R^{13}$ taken together with the atoms through which they are connected form a benzimidazole ring, optionally substituted. In a further detailed aspect, the compound is 5-[5-(6,7-dimethyl-3-oxo-benzo[4,5]imidazo[2,1-b]thiazol-2-ylidenemethyl)-furan-2-yl]-2-hydroxy-benzoic acid.

In a further disclosure the method provides a compound wherein,
$R^6$ is

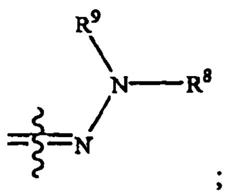

$R^8$ and $R^9$ taken together with the nitrogen atom to which they are attached form a five to eight membered heteroaryl ring having from one to four O, N or S heteroatoms, optionally substituted with benzyl. In a detailed aspect, the compound is 2-hydroxy-5-(5-{2-[2-(5-phenyl-tetrazol-2-yl)-acetylamino]-vinyl}-furan-2-yl)benzoic acid.

In further disclosures the method provides a compound wherein,
$R^6$ is

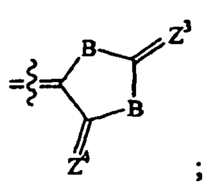

B is independently S or NH; $Z^3$ and $Z^4$ are O. In a detailed aspect, the compound is 5-[5-(2,4-dioxo-thiazolidin-5-ylidenemethyl)-furan-2-yl]-2-hydroxy-benzoic acid.

In further disclosures the method provides a compound wherein,
$R^6$ is

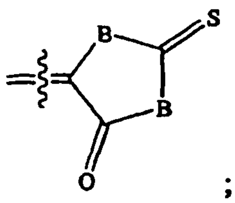

and wherein B is independently S or $NR^{12}$, and wherein $R^{12}$ is N-phenethylcarbamoylmethyl. In a detailed aspect, the compound is 2-hydroxy-4-{5-[4-oxo-3-(phenethylcarbamoyl-methyl)-2-thioxo-thiazofidin-5-ylidenemethyl]-furan-2-yl}-benzoic acid. In a further detailed aspect, the compound is 2-hydroxy-5-{5-[4-oxo-3-(phenethylcarbamoyl-methyl)-2-thioxo-thiazolidin-5-ylidenemethyl]-furan-2-yl}-benzoic acid

In another disclosure a compound is provided of formula:

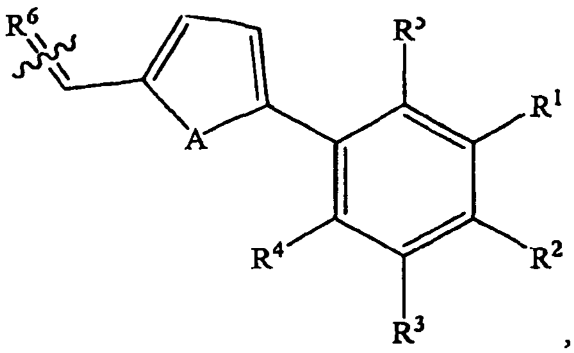

wherein:

A is O, NH or S;
$R^1$, $R^2$, and $R^3$ are each independently H, -OH, $-OR^7$, $-NO_2$, or -(C=O)OH, provided that at least one of $R^1$, $R^2$, and $R^3$ is -OH, $-NO_2$, or -(C=O)OH;
$R^4$ and $R^5$ are each independently H, -OH, $C_1$-$C_6$ alkyl or halo;
$R^6$ is:

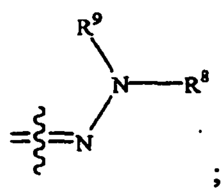

$R^8$ and $R^9$ are each independently H, alkyl, aralkyl, alkanoyl, aralkanoyl or heteroaralkanoyl, or $R^8$ and $R^9$ taken together with the nitrogen atom to which they are attached form a five to eight membered heteroaryl ring having from one to four O, N or S heteroatoms, wherein the heteroaryl ring is optionally substituted.

In further disclosures of the compound,
$R^6$ is

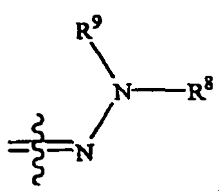

$R^8$ and $R^9$ taken together with the nitrogen atom to which they are attached form a five to eight membered heteroaryl ring having from one to four O, N or S heteroatoms, optionally substituted with benzyl. In a detailed aspect, the compound is 2-hydroxy-5-(5-{2-[2-(5-phenyl-tetrazol-2-yl)-acetylamino]-vinyl}-furan-2-yl)-benzoic acid.

A method of inhibiting *Yersinia* spp. protein tyrosine phosphatase comprises the step of administering to a subject an effective amount of the compound of the present invention. A pharmaceutical composition comprises at least one pharmaceutically acceptable carrier or excipient and an effective amount of the compound of the present invention. A method of inhibiting infection by *Yersinia* spp. comprises the step of administering to the subject an effective amount of the composition of the present invention.

## BRIEF DESCRIPTION OF THE DRAWINGS

Figures 1A, 1B, 1C, and 1D: Lineweaver-Burk plots for 4 inhibitors of *Y. pestis* protein tyrosine phosphatase (YopH). (A) Compound 5557271; (B) Compound 5670901; (C) Compound 5680029; (D) Compound 5842540.

Figures 2A, 2B, 2C, 2D, 2E, 2F, 2G, and 2H: Virtual docking study of binding of YopH inhibitors to protein tyrosine phosphatase

Figures 3A, 3B, 3C, 3D, 3E, and 3F: Molecular model comparison of *Y. pestis* protein tyrosine phosphatase (YopH) with PTP1B and VHR protein tyrosine phosphatases.

Figure 4: Synthetic pathway of YopH inhibitors of protein tyrosine phosphatase.

## DETAILED DESCRIPTION

To avoid detection and targeting by the immune system, the plague-causing bacterium *Yersinia* spp., *e.g., Yersinia pestis,* uses a type III secretion system to deliver a set of inhibitory proteins into the cytoplasm of immune cells. A protein with an active protein tyrosine phosphatase (PTPase) activity, for example, YopH, can paralyze lymphocytes and macrophages by dephosphorylating critical tyrosine kinases and signal transduction molecules. Since *Yersinia pestis* strains lacking YopH are essentially harmless, small-molecule inhibitors for YopH have been developed using chemical library screening, structure-activity relationship analysis, and in silico docking using the three-dimensional structure of the catalytic domain of YopH. A series of furanyl salicylate derivatives selectively inhibit YopH *in vitro* and in YopH-expressing T cells at nanomolar concentrations. Computational docking studies on the most potent inhibitors provide a rationale for the observed inhibitory properties of the compositions which are YopH protein tyrosine phosphatase (PTPase) inhibitors of the present invention. In T cells expressing YopH, the YopH inhibitors restored normal levels of tyrosine phosphorylation and T cell receptor signaling, while control T cells were unaffected.

With respect to a protein tyrosine phosphatase inhibitor, "derivative" refers to a compound of the general formula:

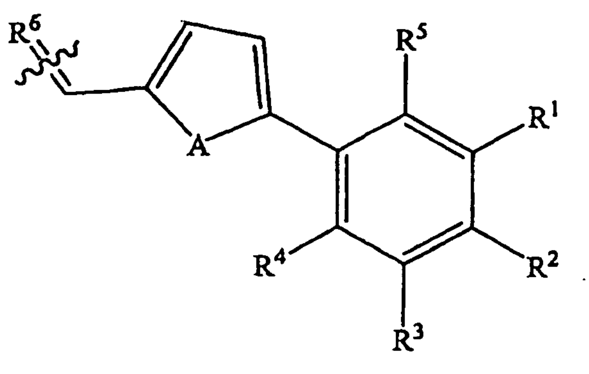

where the variables are as defined herein.

With respect to protein tyrosine phosphatase inhibitor, "analog" or "functional analog" refers to a modified form of the respective protein tyrosine phosphatase inhibitor derivative in which one or more chemically derivatized functional side (*e.g.*, $R_1$, $R_2$, $R_3$, $R_4$, $R_5$, $R_6$, $R_7$, $R_8$, $R_9$, $R_{10}$, $R_{11}$, $R_{12}$, or $R_{13}$; $Z_1$, $Z_2$, $Z_3$, $Z_4$, $Z_5$, $Z_6$, or $Z_7$) or linking groups (*e.g.*, A or B) has been modified such that the analog retains substantially the same biological activity or improved biological activity as the unmodified protein tyrosine phosphatase inhibitor derivative *in vivo* and/or *in vitro.*

"Antagonist" or "protein tyrosine phosphatase antagonist" refers to an endogenous or exogenous compound, substance or entity that opposes the physiological effects of another compound. It can be an endogenous or exogenous compound, substance or entity that has affinity for and opposes and/or blocks at least one of the normal physiological responses normal induced by another compound, substance or entity at the cell receptors. As used herein, the term

refers to a protein tyrosine phosphatase inhibitor derivative or analog, a suitable homolog, or a portion thereof, which blocks at least one of the normal actions of protein tyrosine phosphatase. For example, treatment with certain protein tyrosine phosphatase antagonists or protein tyrosine phosphatase inhibitors can treat a disease state in a mammal caused by infection with *Yersinia* spp.

"Receptor" refers to a molecular structure within a cell or on the surface of the cell which is generally characterized by the selective binding of a specific substance. Exemplary receptors include, for example, cell-surface receptors for peptide hormones, neurotransmitters, antigens, complement fragment, and immunoglobulins and cytoplasmic receptors for steroid hormones. Exemplary receptors specifically can recognize and bind a compound acting as a molecular messenger, for example, neurotransmitter, hormone, lymphokine, lectin, or drug.

The compounds disclosed herein can contain an asymmetric carbon atom. Some of the compounds can contain one or more asymmetric centers and can thus give rise to optical isomers and diastereomers. While shown without respect to stereochemistry in the above formulae, the present invention includes such optical isomers and diastereomers, as well as the racemic and resolved, enantiomerically pure R and S stereoisomers, other mixtures of the R and S stereoisomers and pharmaceutically acceptable salts thereof. There is herein disclosed pro-drugs of the compounds described above and pharmaceutically acceptable salts thereof.

As employed above and throughout the disclosure, the following terms, unless otherwise indicated, shall be understood to have the following meanings.

"Alkyl" refers to an optionally substituted, saturated straight, or branched, hydrocarbon having from about 1 to about 10 carbon atoms (and all combinations and subcombinations of ranges and specific numbers of carbon atoms therein), preferably with from about 1 to about 6, more preferably 1 to about 3 carbon atoms. Alkyl groups can be optionally substituted. Alkyl groups include, but are not limited to, methyl, ethyl, n-propyl, isopropyl, n-butyl, isobutyl, t-butyl, n-pentyl, isopentyl, neopentyl, n-hexyl, isohexyl, 3-methylpentyl, 2,2-dimethylbutyl, and 2,3-dimethylbutyl.

"Alkenyl" refers to an optionally substituted alkyl group having from about 2 to about 10 carbon atoms and one or more double bonds (and all combinations and subcombinations of ranges and specific numbers of carbon atoms therein), wherein alkyl is as previously defined.

"Alkynyl" refers to an optionally substituted alkyl group having from about 2 to about 10 carbon atoms and one or more triple bonds (and all combinations and subcombinations of ranges and specific numbers of carbon atoms therein), wherein alkyl is as previously defined.

"Alkoxy" and "alkoxy" refer to an optionally substituted alkyl-O- group wherein alkyl is as previously defined. Exemplary alkoxy and alkoxyl groups include methoxy, ethoxy, n-propoxy, i-propoxy, n-butoxy, and heptoxy.

"Aryl" and "aromatic" each refer to an optionally substituted, mono-, di-, tri-, or other multicyclic aromatic ring system having from about 5 to about 50 carbon atoms (and all combinations and subcombinations of ranges and specific numbers of carbon atoms therein), with from about 6 to about 10 carbons being preferred. Non-limiting examples include, for example, phenyl, naphthyl, anthracenyl, and phenanthrenyl.

"Aralkyl" refers to an optionally substituted moiety composed of an alkyl radical bearing an aryl substituent and having from about 6 to about 50 carbon atoms (and all combinations and subcombinations of ranges and specific numbers of carbon atoms therein), with from about 6 to about 10 carbon atoms being preferred. Non-limiting examples include, for example, benzyl, diphenylmethyl, triphenylmethyl, phenylethyl, and diphenylethyl.

"Heteroaryl" refers to an optionally substituted aryl ring system wherein in at least one of the rings, one or more of the carbon atom ring members is independently replaced by a heteroatom group selected from the group consisting of S, O, N, and NH, wherein aryl is as previously defined. Heteroaryl groups having a total of from about 5 to about 14 carbon atom ring members and heteroatom ring members(and all combinations and subcombinations of ranges and specific numbers of carbon and heteroatom ring members) are preferred. Exemplary heteroaryl groups include, but are not limited to, pyrryl, furyl, pyridyl, pyridine-N-oxide, 1,2,4-thiadiazolyl, pyrimidyl, thienyl, isothiazolyl, imidazolyl, tetrazolyl, pyrazinyl, pyrimidyl, quinolyl, isoquinolyl, thiophenyl, benzothienyl, isobenzofuryl, pyrazolyl, indolyl, purinyl, carbazolyl, benzimidazolyl, and isoxazolyl. Heteroaryl may be attached via a carbon or a heteroatom to the rest of the molecule.

"Cycloalkyl" or "carbocyclic ring " each refers to an optionally substituted, mono-, di-, tri-, or other multicyclic alicyclic ring system having from about 3 to about 20 carbon atoms (and all combinations and subcombinations of ranges and specific numbers of carbon atoms therein). In some preferred embodiments, the cycloalkyl groups have from about 3 to about 8 carbon atoms. Multi-ring structures may be bridged or fused ring structures, wherein the additional groups fused or bridged to the cycloalkyl ring may include optionally substituted cycloalkyl, aryl, heterocycloalkyl, or heteroaryl rings. Exemplary cycloalkyl groups include, but are not limited to, cyclopropyl, cyclobutyl, cyclopentyl, cyclohexyl, cyclooctyl, adamantyl, 2-[4-isopropyl-1-methyl-7-oxa-bicyclo[2.2.1]heptanyl], and 2-[1,2,3,4-tetrahydro-naphthalenyl].

As used herein, "bicycloalkyl" refers to an optionally substituted, alicyclic group having two bridged rings in its structure and having from about 7 to about 20 carbon atoms (and all combinations and subcombinations of ranges and specific numbers of carbon atoms therein), with from about 7 to about 15 carbon atoms being preferred. Exemplary bicycloalkyl-ring structures include, but are not limited to, norbornyl, bornyl, [2.2.2]-bicyclooctyl, cis-pinanyl, trans-pinanyl, camphanyl, iso-bornyl, and fenchyl.

"Tricycloalkyl" refers to an optionally substituted, alicyclic group having three bridged rings in its structure and having from about 7 to about 20 carbon atoms (and all combinations and subcombinations of ranges and specific numbers of carbon atoms therein), with from about 7 to about 15 carbon atoms being preferred. Exemplary tricycloalkyl-ring structures include, but are not limited to, tricyclo[5.1.2.02,6]decane, 1,7,7-trimethyl tricyclo[2.2.1.02,6]heptane, alpha-santalol, patchouli alcohol, alpha-cedrene, and longifolene.

"Alkylcycloalkyl" refers to an optionally substituted ring system comprising a cycloalkyl group having one or more alkyl substituents, wherein cycloalkyl and alkyl are each as previously defined. Exemplary alkylcycloalkyl groups include, for example, 2-methylcyclohexyl, 3,3-dimethylcyclopentyl, trans-2,3-dimethylcyclooctyl, and 4-methyldecahydronaphthalenyl.

"Cycloalkylalkyl" refers to an optionally substituted ring system composed of an alkyl radical having one or more cycloalkyl substituents, wherein cycloalkyl and alkyl are as previously defined. In some disclosures the alkyl moieties of the cycloalkylalkyl groups have from about 1 to about 3 carbon atoms. Exemplary cycloalkylalkyl groups include, but are not limited to, cyclohexylmethyl, 4-[4-methyldecahydronaphthalenyl]-pentyl, 3-[trans-2,3-dimethylcyclooctyl]-propyl, and cyclopentylethyl.

"Heteroaralkyl" and "heteroarylalkyl" each refers to an optionally substituted ring system composed of a heteroaryl substituted alkyl radical where heteroaryl and alkyl are as previously defined. Non-limiting examples include, for example, 2-(1H-pyrrol-3-yl)ethyl, 3-pyridylmethyl, 5-(2H-tetrazolyl)methyl, and 3-(pyrimidin-2-yl)-2-methylcyclopentanyl.

"Heterocycloalkyl" and "heterocyclic ring" each refers to an optionally substituted ring system composed of a cycloalkyl radical wherein in at least one of the rings, one or more of the carbon atom ring members is independently replaced by a heteroatom group selected from the group consisting of O, S, N, and NH, wherein cycloalkyl is as previously defined. Heterocycloalkyl ring systems having a total of from about 5 to about 14 carbon atom ring members and heteroatom ring members (and all combinations and subcombinations of ranges and specific numbers of carbon and heteroatom ring members) are preferred. In other disclosures the heterocyclic groups may be fused to one or more aromatic rings. Heterocycloalkyl may be attached via a ring carbon or a ring heteroatom to the rest of the molecule. Exemplary heterocycloalkyl groups include, but are not limited to, azepanyl, tetrahydrofuranyl, hexahydropyrimidinyl, tetrahydrothienyl, piperidinyl, pyrrolidinyl, isoxazolidinyl, isothiazolidinyl, pyrazolidinyl, oxazolidinyl, thiazolidinyl, piperazinyl, 2-oxo-morpholinyl, morpholinyl, 2-oxo-piperidinyl, piperadinyl, decahydroquinolyl, octahydrochromenyl, octahydro-cyclopenta[c]pyranyl, 1,2,3,4,-tetrahydroquinolyl, 1,2,3,4-tetrahydroquinazolinyl, octahydro-[2]pyridinyl, decahydro-cycloocta[c]furanyl, 1,2,3,4-tetrahydroisoquinolyl, 2-oxo-imidazolidinyl, and imidazolidinyl.

"Heterocycloalkylalkyl" refers to an optionally substituted ring system composed of an alkyl radical having one or more heterocycloalkyl substituents, wherein heterocycloalkyl and alkyl are as previously defined. In some disclosures the alkyl moieties of the heterocycloalkylalkyl groups have from about 1 to about 3 carbon atoms. Exemplary heterocycloalkyl groups include, but are not limited to, azepanylmethyl, tetrahydrofuranylethyl, hexahydropyrimidinylisobutyl, tetrahydrothienylpropyl, piperidinyl-2,2-dimethylethyl, pyrrolidinylmethyl , isoxazolidinylethyl, isothiazolidinylpropyl, pyrazolidinylmethyl, oxazolidinylbutyl, thiazolidinylisopropyl, piperazinylmethyl, 2-oxo-morpholinylmethyl, morpholinylethyl, 2-oxo-piperidinylethyl, piperadinylmethyl, decahydroquinolylethyl, octahydrochromenylpropyl, octahydro-cyclopenta[c]pyranylbutyl, 1,2,3,4,-tetrahydroquinolylethyl, 1,2,3,4-tetrahydroquinazolinylmethyl, octahydro-[2]pyridinylethyl, decahydro-cycloocta[c]furanylmethyl, 1,2,3,4-tetrahydroisoquinolylmethyl, 2-oxo-imidazolidinylethyl, and imidazolidinylmethyl.

"Spiroalkyl" refers to an optionally substituted, alkylene diradical, both ends of which are bonded to the same carbon atom of the parent group to form a spirocyclic group. The alkylene diradical, taken together with the carbon atom of the parent group to which it is bonded , as herein defined, has 3 to 20 ring atoms, with from 3 to 10 ring atoms being preferred. Exemplary spiroalkyl groups taken together with its parent group include, but are not limited to, 1-(1-methyl-cyclopropyl)-propan-2-one, 2-(1-phenoxy-cyclopropyl)-ethylamine, and 1-methyl-spiro[4.7]dodecane.

"Halo" and "halogen" each refers to a fluoro, chloro, bromo, or iodo moiety with fluoro, chloro, or bromo moieties being preferred:

"Perfluorinated", when used in conjunction with "alkyl" refers to an alkyl group wherein the hydrogen atoms attached to the terminal carbon of the alkyl chain are replaced by fluorine atoms, more preferably the hydrogen atoms attached to the terminal carbon and one or more of the remaining hydrogen atoms attached to the alkyl chain are replaced by fluorine atoms, with all hydrogen atoms attached to the alkyl chain being replaced by fluorine atoms being most preferred, and wherein alkyl is as previously defined.

Typically, substituted chemical moieties include one or more substituents that replace hydrogen. Exemplary substituents include, for example, halo (*e.g.*, F, Cl, Br, I), alkyl, cycloalkyl, alkylcycloalkyl, alkenyl, alkynyl, aralkyl, aryl, heteroaryl, heteroaralkyl, spiroalkyl, heterocycloalkyl, hydroxyl (-OH), oxo (=O), nitro (-NO2), cyano (-CN), amino (-NH2), -N-substituted amino (-NHR''), -N,N-disubstituted amino (-N(R'')R''), carboxy (-COOH), -O-C(=O)R'', -C(=O)R'', -OR'', -C(=O)OR'', -NHC(=O)R'', aminocarbonyl (-C(=O)NH2), -N-substituted aminocarbonyl (-C(=O)NHR''), -N,N-disubstituted aminocarbonyl (-C(=O)N(R')R''), thiol, thiolato (-SR''), sulfonic acid (-SO3H phosphonic acid (-PO3H), -P(=O)(OR'')OR'', S(=O)R'', -S(=O)2R'', -S(=O)2NH2, -S(=O)2 NHR'', -S(=O)2NR''R'', -NHS(=O)2R'', NR''S(=O)2R'', -CF3,

-CF2CF3, -NHC(=O)NHR", -NHC(=O)NR"R", -NR"C(=O)NHR", -NR"C(=O)NR"R", NR"C(=O)R" and the like. In relation to the aforementioned substituents, each moiety R" can be, independently, any of H, alkyl, cycloalkyl, alkenyl, aryl, aralkyl, heteroaryl, or heterocycloalkyl, or when two R" groups are attached to the same nitrogen atom within a substituent, as herein above defined, R" and R" can be taken together with the nitrogen atom to which they are attached to form a 3- to 8-membered heterocycloalkyl ring, wherein one or two of the heterocycloalkyl ring carbon atoms independently may be optionally replaced by-0-, -S-, -SO, -SO2-, -NH-, -N(alkyl)-, -N(acyl)-, -N(aryl)-, or -N(aroyl)- groups, for example.

"Pharmaceutically acceptable" refers to a substance that is acceptable for use in pharmaceutical applications from a toxicological perspective and does not adversely interact with the active ingredient.

"Substituted" refers to a moiety, such as an aryl, heteroaryl, cycloalkyl or heterocycloalkyl moiety having from 1 to about 5 substituents, and more preferably from 1 to about 3 substituents independently selected from a halo, a cyano, nitro or hydroxyl group, a $C_1$ to $C_6$ alkyl group, or a $C_1$ to $C_6$ alkoxy group. Preferred substituents are a halo, a hydroxyl group, or a $C_1$ to $C_6$ alkyl group.

"Optional" or "optionally" means that the subsequently described event or circumstance may or may not occur, and that the description includes instances in which it does not. For example, optionally substituted phenyl indicates either unsubstituted phenyl, or phenyl mono-,di-, or tri-substituted, independently, with OH, COOH, lower alkyl, lower alkoxy, halo, nitro, amino, alkylamino, dialkylamino, trifluoromethyl and/or cyano.

"Effective amount" refers to an amount of a compound that can be therapeutically effective to inhibit, prevent or treat the symptoms of particular disease, disorder or side effect.

"Pharmaceutically acceptable" refers to those compounds, materials, compositions, and/or dosage, forms which are, within the scope of sound medical judgment, suitable for contact with the tissues of human beings and animals without excessive toxicity, irritation, allergic response, or other problem complications commensurate with a reasonable benefit/risk ratio.

"In combination with", "combination therapy" and "combination products" refer, in certain embodiments, to the concurrent administration to a patient of a first therapeutic and the compounds as used herein. When administered in combination, each component can be administered at the same time or sequentially in any order at different points in time. Thus, each component can be administered separately but sufficiently closely in time so as to provide the desired therapeutic effect.

"Dosage unit" refers to physically discrete units suited as unitary dosages for the particular individual to be treated. Each unit can contain a predetermined quantity of active compound(s) calculated to produce the desired therapeutic effect(s) in association with the required pharmaceutical carrier. The specification for the dosage unit forms can be dictated by (a) the unique characteristics of the active compound(s) and the particular therapeutic effect(s) to be achieved, and (b) the limitations inherent in the art of compounding such active compound(s).

"Hydrate" refers to a compound of the present invention which is associated with water in the molecular form, i.e., in which the H-OH bond is not split, and may be represented, for example, by the formula $R \cdot H_2O$, where R is a compound of the invention. A given compound may form more than one hydrate including, for example, monohydrates ($R \cdot H_2O$), or polyhydrates ($R \cdot nH_2O$ wherein n is an integer > 1) including, for example, dihydrates ($R \cdot 2H_2O$), trihydrates ($R \cdot 3H_2O$), and the like, or hemihydrates, such as, for example, $R \cdot n/2H_2O$, $R \cdot n/3H_2O$, $R \cdot n/4H_2O$ and the like wherein n is an integer.

"Solvate" refers to a compound of the present invention which is associated with solvent in the molecular form, i.e., in which the solvent is coordinatively bound, and may be represented, for example, by the formula R·(solvent), where R is a compound of the invention. A given compound may form more than one solvate including, for example, monosolvates (R·(solvent)) or polysolvates (R·n(solvent)) wherein n is an integer > 1) including, for example, disolvates (R·2(solvent)), trisolvates (R·3(solvent)), and the like, or hemisolvates, such as, for example, R·n/2(solvent), R·n/3 (solvent), R·n/4(solvent) and the like wherein n is an integer. Solvents herein include mixed solvents, for example, methanol/water, and as such, the solvates may incorporate one or more solvents within the solvate.

"Acid hydrate" refers to a complex that may be formed through association of a compound having one or more base moieties with at least one compound having one or more acid moieties or through association of a compound having one or more acid moieties with at least one compound having one or more base moieties, said complex being further associated with water molecules so as to form a hydrate, wherein said hydrate is as previously defined and R represents the complex herein described above.

"Pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorgainc or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic,

glutamic, benzoic, salicylic, sulfanilic, 2-acetoxybenzoic, fumaric, toluenesulfonic, methanesulfonic, ethane disulfonic, oxalic, isethionic, and the like. These physiologically acceptable salts are prepared by methods known in the art, e.g., by dissolving the free amine bases with an excess of the acid in aqueous alcohol, or neutralizing a free carboxylic acid with an alkali metal base such as a hydroxide, or with an amine.

Compounds described herein throughout, can be used or prepared in alternate forms. For example, many amino-containing compounds can be used or prepared as an acid addition salt. Often such salts improve isolation and handling properties of the compound. For example, depending on the reagents, reaction conditions and the like, compounds as described herein can be used or prepared, for example, as their hydrochloride or tosylate salts. Isomorphic crystalline forms, all chiral and racemic forms, N-oxide, hydrates, solvates, and acid salt hydrates, are also contemplated to be within the scope of the present invention.

Certain acidic or basic compounds of the present invention may exist as zwitterions. All forms of the compounds, including free acid, free base and zwitterions, are contemplated to be within the scope of the present invention. It is well known in the art that compounds containing both amino and carboxy groups often exist in equilibrium with their zwitterionic forms. Thus, any of the compounds described herein throughout that contain, for example, both amino and carboxy groups, also include reference to their corresponding zwitterions.

"Patient" refers to animals, including mammals, preferably humans.

"Prodrug" refers to compounds specifically designed to maximize the amount of active species that reaches the desired site of reaction, which are of themselves typically inactive or minimally active for the activity desired, but through biotransformation are converted into biologically active metabolites.

"Stereoisomers" refers to compounds that have identical chemical constitution, but differ as regards the arrangement of the atoms or groups in space.

"Partial stereoisomer" refers to stereoisomers having two or more chiral centers wherein at least one of the chiral centers has defined stereochemistry (i.e., R or S) and at least one has undefined stereochemistry (i.e., R or S). When the term "partial stereoisomers thereof" is used herein, it refers to any compound within the described genus whose configuration at chiral centers with defined stereochemistry centers is maintained and the configuration of each undefined chiral center is independently selected from R or S. For example, if a stereoisomer has three chiral centers and the stereochemical configuration of the first center is defined as having "S" stereochemistry, the term "or partial stereoisomer thereof" refers to stereoisomers having SRR, SRS, SSR, or SSS configurations at the three chiral centers, and mixtures thereof.

"N-oxide" refers to compounds wherein the basic nitrogen atom of either a heteroaromatic ring or tertiary amine is oxidized to give a quaternary nitrogen bearing a positive formal charge and an attached oxygen atom bearing a negative formal charge.

When any variable occurs more than one time in any constituent or in any formula, its definition in each occurrence is independent of its definition at every other occurrence. Combinations of substituents and/or variables are permissible only if such combinations result in stable compounds.

## METHODS OF TREATMENT

A method for treatment of a disease state in a mammal infected with *Yersinia* spp. comprises administering to the mammal a therapeutic amount of a compound, which is an inhibitor of a *Yersinia* spp. protein tyrosine phosphatase (PTPase) of the present invention.

A method for treatment of a disease state in a mammal infected with *Yersinia* spp. comprises administering a therapeutic amount of an inhibitor of the *Yersinia* spp YopH protein, a protein tyrosine phosphatase (PTPase). YopH is a 468-amino acid, active PTPase with a C-terminal catalytic domain and a multifunctional N-terminal domain, which binds tyrosine-phosphorylated target proteins. Guan, et al., Science 249: 553-556, 1990; Bliska, et al., Proc. Natl. Acad. Sci USA 88: 1187-1191, 1991; Montagna, et al., J. Biol. Chem. 276: 5 005-5011, 2001; Evdokimov, et al., Acta Cryst. 57: 793-799, 2001. The catalytic domain of YopH is structurally very similar to that of eukaryotic PTPases. Stuckey, et al., Nature 370: 571-575, 1994. A marked dephosphorylation of proteins in human epithelial cells and murine macrophages has been observed during infection with live bacteria. Andersson, et al., Mol. Microbiol. 20: 1057-1069, 1996; Bliska, et al., Proc. Natl. Acad. Sci USA 88: 1187-1191, 1991; Bliska, et al., J. Exp. Med. 176: 1625-1630, 1992; Hartland, et al., Infect. Immun. 62: 4445-4453, 1994. In macrophages and neutrophils, the dephosphorylated proteins include the focal adhesion proteins Cas, focal adhesion kinase, and paxillin, providing a molecular mechanism for inhibition of migration and phagocytosis by these cells. Persson, et al., EMBO J. 16: 2307-2318; 1997; Black, et al., EMBO J. 16: 2730-2744, 1997; Black, et al., Mol. Microbiol. 29: 1263-1274, 1998; Ruckdeschel, et al., Infect. Immun. 64: 724-733, 1996.

YopH also inhibits the activation of T and B lymphocytes. Yao, et al., J. Exp. Med. 190: 1343-1350, 1999. YopH, transfected into T cells and expressing the YopH PTPase, directly dephosphorylated the Src family tyrosine kinase Lck at its positive regulatory site, Y394, resulting in a complete loss of Lck activity. Since this kinase is the first upstream signal generating molecule for the T cell antigen receptor, signaling from this receptor was completely abrogated. As a consequence, all tyrosine phosphorylation of downstream signaling proteins was inhibited, the T cells failed to form immune synapses with antigen-presenting cells, and they were unable to secrete any interleukin-2 into the medium. Alonso, et aL, J. Biol. Chem. 279: 4922-4928, 2004. Similarly, T cells exposed to live *Yersinia enterocolitica* became unable to flux calcium and produce cytokines. Sauvonnet, et al., Mol. Microbiol. 45: 805-815, 2002.

Since *Yersinia* strains that carry a pYV plasmid with a nonfunctional *yopH* gene are avirulent and even a point-mutation that changes the catalytic Cys-403 to an alanine eliminates the virulence of *Yersinia pseudotuberculosis* in a murine infection model, it is clear that the catalytic activity of YopH is a necessary factor for the lethality of *Yersinia* infection. Bliska, et al., Proc. Natl. Acad. Sci US4 88: 1187-1191, 1991; Bliska, et at., J. Exp. Med. 176: 1625-1630, 1992; Bolin, et al., Mol. Microbiol. 2: 237-245, 1988; Michielis, et al., Microbial. Pathogen 5: 449-459,1988; Rosqvist, et al., Infect. Immun. 56: 2139-2143, 1988; Straley, et al., Infect. Immun. 51: 445-454, 1986

Methods and compositions of the present invention have identified and isolated small-molecule inhibitors of YopH (PTPase) by a combination of chemical library screening, structure-activity analysis, and *in silico* docking of lead compounds. Methods and compositions of the present invention, comprise small molecule inhibitors, *e.g*., furanyl-salicylate derivatives, that are inhibitors of the PTPase activity of YopH. The PTPase activity of YopH is essential for the virulence of *Yersinia pestis,* the causative agent of plague. The YopH inhibitors contain, for example, a single salicylate linked to a furanyl moiety and a more variable group. In a detailed embodiment, since the YopH inhibitors of the present invention have only one carboxylic group, they can penetrate into lymphocytes and reverse the inhibitory effects of YopH on T cell antigen receptor signaling. These results demonstrate that selective and potent YopH inhibitors can be developed to combat the virulence of *Yersinia pestis.* Particularly in the case of multidrug resistant strains or following exposure to aerosolized *Y. pestis,* the YopH inhibitors of the present invention can prove useful to treat *Y. pestis* infection.

The approach to isolating and identifying YopH inhibitors (PTPase inhibitors) of the present invention can be characterized as a hybrid approach between traditional high-throughput screening and a rational design with the substrate of an enzyme as a starting point. High-throughput screening of chemical entities was used only to identify useful lead structures, which then were taken into *in silico* docking studies as the main platform, on which the inhibitory properties of the salicylate-furanyl compounds, as potential YopH inhibitors, were examined at the atomic level. This approach provided a detailed insight into the complex network of hydrogen bonds between the enzyme and the inhibitors and thus provided an understanding of the experimental results with analogs of the first hits. This approach, in turn, made it possible to rationally design even better inhibitors. The docking studies showed that the salicylate moiety ('ring A') mimics the phosphotyrosine residue of a substrate for YopH, and the salicylate moiety ('ring A') fits into the catalytic pocket (P1). The furanyl ring ('ring B') interacts specifically with Gln357 on the rim of the catalytic pocket and with the side chain of Arg404. Since both these residues are unique to YopH compared to other PTPases, the furanyl ring apparently provides selectivity to the inhibitors. The positioning and the interactions involving ring C were more variable, as seen experimentally by a higher tolerance for substitutions at this position. However, ring C can bridge the core salicylate-furanyl structure occupying the catalytic pocket ('P1') to two other unique pockets present on the surface of YopH, termed 'P2' and 'P3' (Fig. 3). The relative distances between ring C and the edge of each cavity vary from 1.5 - 13 Å in the best solutions of the docked structures. This short distance suggests the possibility that derivatives of YopH (PTPase) inhibitors that reach into P2 or P3 can have increased affinity and selectivity for YopH. In fact, a comparison of the surface of YopH to that of two other crystallized PTPases, such as PTP1B and VHR, reveals that these additional sub-pockets are either different in nature or absent in these proteins (Fig.. 3). Indeed, the entire topology of the surface that surrounds the catalytic pocket is dramatically different between these three PTPases: in YopH there is a large semicircular valley bordered by the three pockets and includes a low ridge between P2 and P3 giving the valley a V-shaped floor. In PTP1B there are only two pockets and the substrate binding depression is elongated and constricted into a narrow passage between P1 and P2 (Fig. 3 C and D). In VHR, there is only one pocket surrounded by several negatively charged peaks. These striking differences in surface topology provide increased confidence in a rational design of selective inhibitors for PTPases. These differences probably also reflect, at least in part, differences in substrate selection by PTPases in their cellular environments. However, pockets P2 and P3 are not occupied when YopH is complexed with a short non-hydrolyzable peptide, Ac-DADE-$F_2$Pmp-L-$NH_2$. In this complex, the peptide binds in a manner that is similar to the way in which a corresponding hexapeptide binds to human PTP1B. Salmeen et al., Mol. Cell. 6: 1401-1412, 2000. Thus, small molecule inhibitors can be designed to utilize surface features of PTPases beyond what is involved in substrate interaction. The methods and compositions of the present invention, YopH inhibitors, are synthesized as bi-dentated compounds that also occupy P2 or P3, while keeping molecular weight, number of rotatable bonds and ClogP (partition coefficient) in the new compounds below 500-600, 8 and ~2.5, respectively, to achieve compounds with most favorable "drug-like" properties. The methods and compositions of the present invention, YopH inhibitors, are optimal for such further modifications.

## Detection of Protein Tyrosine Phosphatase Inhibitor Derivatives and Analogs

Protein tyrosine phosphatase inhibitor derivatives and analogs can be detected and quantified by any of a

number of means well known to those of skill in the art. These include analytic biochemical methods such as electrophoresis, capillary electrophoresis, high performance liquid chromatography (HPLC), mass spectrometry, thin layer chromatography (TLC), hyperdiffusion chromatography, and the like, or various immunological methods such as fluid or gel precipitin reactions, immunodiffusion (single or double), immunoelectrophoresis, radioimmunoassay (RIA), enzyme-linked immunosorbent assays (ELISAs), immunofluorescent assays, western blotting, and the like.

In one approach protein tyrosine phosphatase inhibitor derivatives and analogs are detected using an immunoassay such as an ELISA assay (*see, e.g.,* Crowther, John R. ELISA Theory and Practice. Humana Press: New Jersey, 1995). An "immunoassay" is an assay that utilizes an antibody to specifically bind to a protein tyrosine phosphatase inhibitor derivatives and analogs.

## PHARMACEUTICAL COMPOSITIONS

Protein tyrosine phosphatase inhibitor derivatives and analogs useful in the present compositions and methods can be administered to a human patient *per se,* in the form of a stereoisomer, prodrug, pharmaceutically acceptable salt, hydrate, solvate, acid salt hydrate, N-oxide or isomorphic crystalline form thereof, or in the form of a pharmaceutical composition where the compound is mixed with suitable carriers or excipient(s) in a therapeutically effective amount, for example, heart disease or congestive heart failure.

### Routes of Administration

The protein Tyrosine phosphatase inhibitor derivatives and analogs and pharmaceutical compositions described herein can be administered by a variety of routes. Suitable routes of administration can, for example, include oral, rectal, transmucosal, or intestinal administration, parenteral delivery, including intramuscular, subcutaneous, intramedullary injection, as well as intrathecal, direct intraventricular, intravenous, intraperitoneal, spinal, epidural, intranasal, or intraocular injections. Alternatively, one can administer the compound in a local rather than systemic manner, for example via injection of the compound directly into the subject, often in a depot or sustained release formulation. Furthermore, one can administer the compound in a targeted drug delivery system, for example, in a liposome coated vesicle. The liposomes can be targeted to and taken up selectively by the tissue of choice. In a further disclosure the protein tyrosine phosphatase inhibitor derivatives and analogs and pharmaceutical compositions described herein are administered orally.

### Composition/Formulation

The pharmaceutical compositions described herein can be manufactured in a manner that is itself known, *e.g.*, by means of conventional mixing, dissolving, granulating, dragee-making, levigating, emulsifying, encapsulating, entrapping or lyophilizing processes. Pharmaceutical compositions for use as described herein can be formulated in conventional manner using one or more physiologically acceptable carriers comprising excipients and auxiliaries which facilitate processing of the active compounds into preparations which can be used pharmaceutically. Proper formulation is dependent upon the route of administration chosen. For injection, the agents can be formulated in aqueous solutions, *e.g.*, in physiologically compatible buffers such as Hanks' solution, Ringer's solution, or physiological saline buffer. For transmucosal administration, penetrants appropriate to the barrier to be permeated are used in the formulation. Such penetrants are generally known in the art. For oral administration, the compounds can be formulated readily by combining with pharmaceutically acceptable carriers that are well known in the art. Such carriers enable the compounds to be formulated as tablets, pills, dragees, capsules, liquids, gels, syrups, slurries, suspensions and the like, for oral ingestion by a patient to be treated. Pharmaceutical preparations for oral use can be obtained by mixing the compounds with a solid excipient, optionally grinding a resulting mixture, and processing the mixture of granules, after adding suitable auxiliaries, if desired, to obtain tablets or dragee cores.

Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents can be added, such as the cross-linked polyvinyl pyrrolidone, agar, or alginic acid or a salt thereof such as sodium alginate. Dragee cores are provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain

the active ingredients in admixture with filler such as lactose, binders such as starches, and/or lubricants such as talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. All formulations for oral administration should be in dosages suitable for such administration. For buccal administration, the compositions can take the form of tablets or lozenges formulated in conventional manner. For administration by inhalation, the compounds for use are conveniently delivered in the form of an aerosol spray presentation from pressurized packs or a nebuliser, with the use of a suitable propellant, *e.g.*, dichlorodifluoromethane, trichlorofluoromethane, dichlorotetrafluoroethane, carbon dioxide or other suitable gas. In the case of a pressurized aerosol the dosage unit can be determined by providing a valve to deliver a metered amount. Capsules and cartridges of *e.g.*. gelatin for use in an inhaler or insufflator can be formulated containing a powder mix of the compound and a suitable powder base such as lactose or starch.

The compounds can be formulated for parenteral administration by injection, *e.g.*, by bolus injection or continuous infusion. Formulations for injection can be presented in unit dosage form, *e.g.*, in ampules or in multi-dose containers, with an added preservative. The compositions can take such forms as suspensions, solutions or emulsions in oily or aqueous vehicles, and can contain formulatory agents such as suspending, stabilizing and/or dispersing agents. Pharmaceutical formulations for parenteral administration include aqueous solutions of the active compounds in water-soluble form. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions can contain substances which increase the viscosity of the suspension, such as sodium carboxymethyl cellulose, sorbitol, or dextran. Optionally, the suspension can also contain suitable stabilizers or agents which increase the solubility of the compounds to allow for the preparation of highly concentrated solutions. Alternatively, the active ingredient can be in powder form for constitution with a suitable vehicle, *e.g.*, sterile pyrogen-free water, before use.

The compounds can also be formulated in rectal compositions such as suppositories or retention enemas, *e.g.*, containing conventional suppository bases such as cocoa butter or other glycerides. In addition to the formulations described previously, the compounds can also be formulated as a depot preparation. Such long acting formulations can be administered by implantation (for example subcutaneously or intramuscularly) or by intramuscular injection. Thus, for example, the compounds can be formulated with suitable polymeric or hydrophobic materials (for example as an emulsion in an acceptable oil) or ion exchange resins, or as sparingly soluble derivatives, for example, as a sparingly soluble salt.

A suitable pharmaceutical carrier for hydrophobic compounds is a cosolvent system comprising benzyl alcohol, a nonpolar surfactant, a water-miscible organic polymer, and an aqueous phase. The cosolvent system can be the VPD co-solvent system. VPD is a solution of 3% (w/v) benzyl alcohol, 8% (w/v) of the nonpolar surfactant polysorbate 80, and 65% (w/v) polyethylene glycol 300, made up to volume in absolute ethanol. The VPD co-solvent system (VPD:5W) consists of VPD diluted 1:1 with a 5% (w/v) dextrose in water solution. This co-solvent system dissolves hydrophobic compounds well, and itself produces low toxicity upon systemic administration. Naturally, the proportions of a co-solvent system can be varied considerably without destroying its solubility and toxicity characteristics. Furthermore, the identity of the co-solvent components can be varied: for example, other low-toxicity nonpolar surfactants can be used instead of polysorbate 80; the fraction size of polyethylene glycol can be varied; other biocompatible polymers can replace polyethylene glycol, *e.g.* polyvinyl pyrrolidone; and other sugars or polysaccharides can substitute for dextrose. Alternatively, other delivery systems for hydrophobic pharmaceutical compounds can be employed. Liposomes and emulsions are well known examples of delivery vehicles or carriers for hydrophobic drugs. Certain organic solvents such as dimethylsulfoxide also can be employed, although usually at the cost of greater toxicity.

Additionally, the compounds can be delivered using a sustained-release system, such as semipermeable matrices of solid hydrophobic polymers containing the therapeutic agent. Various types of sustained-release materials have been established and are well known by those skilled in the art. Sustained-release capsules can, depending on their chemical nature, release the compounds for a few weeks up to over 100 days. The pharmaceutical compositions also can comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Pharmaceutically acceptable carriers are determined in part by the particular composition being administered, as well as by the particular method used to administer the composition. Accordingly, there is a wide variety of suitable formulations of pharmaceutical compositions for administering the protein tyrosine phosphatase inhibitor *(see, e.g.,* Remington's Phannaceutical Sciences, Mack Publishing Co., Easton, PA 18th ed., 1990, incorporated herein by reference). The pharmaceutical compositions generally comprise a differentially expressed protein, agonist or antagonist in a form suitable for administration to a patient. The pharmaceutical compositions are generally formulated as sterile, substantially isotonic and in full compliance with all Good Manufacturing Practice (GMP) regulations of the U.S. Food and Drug Administration.

**Effective Dosages**

Pharmaceutical compositions suitable for use include compositions wherein the protein tyrosine phosphatase inhibitor derivatives and analogs are contained in a therapeutically effective amount. Determination of an effective amount is well within the capability of those skilled in the art, especially in light of the detailed disclosure provided herein. For any compound used in the present method, a therapeutically effective dose can be estimated initially from cell culture assays. For example, a dose can be formulated in animal models to achieve a circulating concentration range that includes the $I_{50}$ as determined in cell culture (i.e., the concentration of test compound that is lethal to 50% of a cell culture) or the $I_{100}$ as determined in cell culture (i.e., the concentration of compound that is lethal to 100% of a cell culture). Such information can be used to more accurately determine useful doses in humans. Initial dosages can also be formulated by comparing the effectiveness of the protein tyrosine phosphatase inhibitor derivatives and analogs described herein in cell culture assays with the effectiveness of known heart medications. In this method an initial dosage can be obtained by multiplying the ratio of effective concentrations obtained in cell culture assay for the protein tyrosine phosphatase inhibitor derivatives and analogs and a known heart drug by the effective dosage of the known heart drug. For example, if a protein tyrosine phosphatase inhibitor derivative or analog is twice as effective in cell culture assay than the heart drug *(i.e.,* the $I_{50}$ $T_1$ amine is equal to one half times the $I_{50}$ heart drug in the same assay), an initial effective dosage of the protein tyrosine phosphatase inhibitor derivative or analog would be one-half the known dosage for the heart drug. Using these initial guidelines one having ordinary skill in the art could determine an effective dosage in humans. Initial dosages can also be estimated from *in vivo* data. One having ordinary skill in the art could readily optimize administration to humans based on this data. Dosage amount and interval can be adjusted individually to provide plasma levels of the active compound which are sufficient to maintain therapeutic effect. Usual patient dosages for oral administration range from about 50-2000 mg/kg/day, typically from about 250-1000 mg/kg/day, from about 500-700 mg/kg/day or from about 350-550 mg/kg/day. Therapeutically effective serum levels will be achieved by administering multiple doses each day. In cases of local administration or selective uptake; the effective local concentration of the drug can not be related to plasma concentration. One having skill in the art will be able to optimize therapeutically effective local dosages without undue experimentation. The amount of composition administered will, of course, be dependent on the subject being treated, on the subject's weight, the severity of the affliction, the manner of administration and the judgment of the prescribing physician. The therapy can be repeated intermittently while congestive heart failure is detectable or even when they are not detectable. Moreover, due to its apparent nontoxicity, the therapy can be provided alone or in combination with other drugs, such as for example, anti-inflammatories, antibiotics, corticosteroids, vitamins and the like. Possible synergism between the protein tyrosine phosphatase inhibitor derivatives or analogs described herein and other drugs can occur. In addition, possible synergism between a plurality of protein tyrosine phosphatase inhibitor derivatives or analogs can occur.

The typical daily dose of a pharmaceutical composition of protein tyrosine phosphatase inhibitor derivatives and analogs varies according to individual needs, the condition to be treated and with the route of administration. Suitable doses are in the general range of from 0.001 to 10 mg/kg bodyweight of the recipient per day. Within this general dosage range, doses can be chosen at which the pharmaceutical composition of protein tyrosine phosphatase inhibitor derivatives and analogs has an inotropic effect to increase cardiac output without the chronotropic effect to increase heart rate. In general, but not exclusively, such doses will be in the range of from 0.5 to 10 mg/kg.

In addition, within the general dose range, doses can be chosen at which the compounds pharmaceutical composition of protein tyrosine phosphatase inhibitor derivatives and analogs has an inotropic effect to increase cardiac output without the chronotropic effect to increase heart rate. In general, but not exclusively, such doses will be in the range of from 0.001 to 0.5 mg/kg. It is to be understood that the 2 sub ranges noted above are not mutually exclusive and that the particular activity encountered at a particular dose will depend on the nature of the pharmaceutical composition of protein tyrosine phosphatase inhibitor derivatives and analogs used. The pharmaceutical composition of protein tyrosine phosphatase inhibitor derivatives and analogs can be in unit dosage form, for example, a tablet or a capsule so that the patient can self-administer a single dose. In general, unit doses contain in the range of from 0.05-100 mg of a compound of the pharmaceutical composition of protein tyrosine phosphatase inhibitor derivatives and analogs. Unit doses contain from 0.05 to 10 mg of the pharmaceutical composition. The active ingredient can be administered from 1 to 6 times a day. Thus daily doses are in general in the range of from 0.05 to 600 mg per day. In an alternative daily doses are in the range of from 0.05 to 100 mg per day or from 0.05 to 5 mg per day.

Toxicity

Toxicity and therapeutic efficacy of the protein tyrosine phosphatase inhibitor derivatives and analogs described herein can be determined by standard pharmaceutical procedures in cell cultures or experimental animals, *e.g.*, by determining the $LD_{50}$ (the dose lethal to 50% of the population) and the $ED_{50}$ (the dose therapeutically effective in 50% of the population). The dose ratio between toxic and therapeutic effect is the therapeutic index and can be expressed

as the ratio between $LD_{50}$ and **$ED_{50}$** Compounds which exhibit high therapeutic indices are chosen. The data obtained from these cell culture assays and animal studies can be used in formulating a dosage range that is not toxic for use in human. The dosage of such compounds lies within a range of circulating concentrations that include the $ED_{50}$ with little or no toxicity. The dosage can vary within this range depending upon the dosage form employed and the route of administration utilized. The exact formulation, route of administration and dosage can be chosen by the individual physician in view of the patient's condition. (See, *e.g.*, Fingl et al., 1975, In: The Pharmacological Basis of Therapeutics, Ch.1, p.1). One of the advantages, among others, of using the protein tyrosine phosphatase inhibitor derivatives and analogs described herein to treat congestive heart failure is their lack of toxicity. For example, it has been found that repeated intraperitoneal doses of 75mg/kg produced no ill effects in mice (see Example 5). Since the i.v. serum half-life ($t_{1/2}$) of $T_1$amine is about 2-2.5 hours, repeated daily dosages of the protein tyrosine phosphatase inhibitor described herein without ill effects is predictable.

**EXEMPLARY EMBODIMENTS**

**Example 1**

**Identification of Lead Compounds By Chemical Library Screening**

A 96-well format *in vitro* assay was used to screen the first 10,000 compounds of the DIVERSet™ library (ChemBridge Corporation, San Diego, CA.) of drug-like compounds. A total of 10 compounds inhibited YopH to a higher extent than 200 μM orthovanadate, a general PTPase inhibitor. After determination of the kinetic parameters of these 10 first hits, four compounds were selected, which showed a competitive or mixed inhibition pattern with a Ki value <10 μM. Their structures and kinetic data are shown in Table 1.

**Table 1: Kinetic data**

| ID# | Structure | Ki(c) [μM] | Ki(u) [μM] |
|---|---|---|---|
| 5760449 (illustrative) | 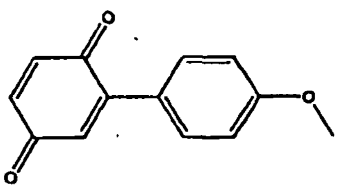 | **6.52 ± 1.19** | 8.02 ± 0.589 |
| 5842540 | 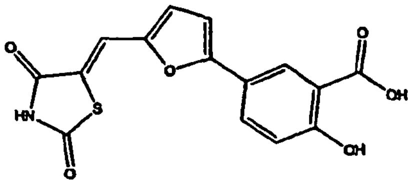 | **1.13 ± 0.198** | 14.1 ± 4.21 |
| 5850330 (illustrative) | 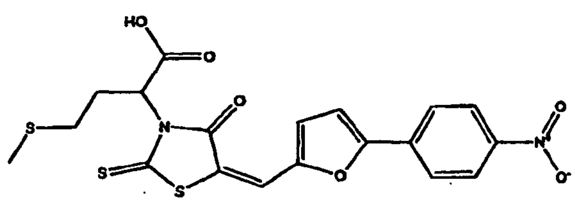 | **9.78 ± 3.32** | |
| 5858065 (illustrative) | 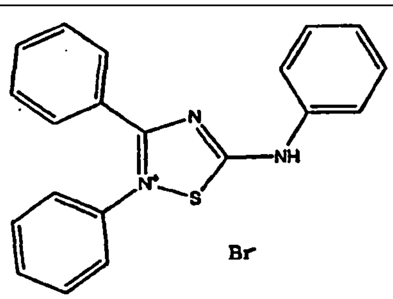 | **1.87 ± 0.924** | 2.57 ± 0.563 |

**Example 2**

**Structure-Activity Relationship Analysis of Initial Inhibitors**

To study the molecular basis for inhibition of YopH, a total of 79 commercially available analogs of our first 4 leads were investigated. ChemBridge Corporation, San Diego, CA. Among the 12 analogs of compound 5760449, only the benzoquinones had an inhibitory effect on YopH, while the naphthoquinone analog of 5760449 did not inhibit the enzyme at all. This suggested that the benzoquinone moiety of 5760449 binds in the catalytic pocket of the phosphatase, mimicking the phosphotyrosine substrate. However, since quinones can oxidize the active site thiol or form covalent adducts, the time-dependence of these inhibitors was measured. Indeed, the most effective analog, 4-(3,6-dioxocyclohexa-1,4-dienyl)benzoic acid, clearly showed a time-dependent inhibition. Therefore quinones were not studied further.

Compound 5858065 has a structure with a charged thiadiazole ring in its core (Table 1). Analogs of this structure were not studied further.

A closer inspection of compounds 5842540 and 5850330 reveals some similarities between them. In compound 5842540, the salicylic acid moiety most likely acts as a phosphotyrosine mimetic. The corresponding structural element in compound 5850330 could be the nitrophenyl ring. In both compounds these moieties are linked via a furanyl ring to a 5-methylenethiazolidine-2,4-dione in compound 5842540, and a 5-methylene-2-thioxothiazolidin-4-one, which is additionally substituted at the nitrogen atom, in compound 5850330. Compounds 5842540 and 5850330 inhibited YopH in a rather selective manner selective (Table 2) prompting further investigation of these inhibitory properties of these series of compounds.

**Table 2: Selectivity of Compounds 5842540 and 5850330** (illustrative)

| | 5842540<br>**IC50 [μM]** | 5850330<br>**IC50 [μM]** |
|---|---|---|
| YopH | **3.9** | **11.5** |
| PTP 1B | **12.0** | **38.8** |
| TCPTP | 30.2 | 197 |
| LAR | **12.2** | **20.8** |
| CD45 | **5.7** | **38.7** |
| VHR | 27.5 | 49.0 |
| VH1 | >250 | >250 |
| VHX | 38.5 | 106 |
| HePTP | 34.5 | 95.9 |
| LMPTP B | 215 | >300 |

**Example 3**

**Structure-Activity Relationship Analysis of 5842540 and 5850330**

Analogs of compounds 5842540 and 5850330 were investigated for the structure-activity relationships. A total of 61 analogs were studied. Each contained a substituted phenyl ring linked via a furanyl moiety and further to a more diverse entity at the other end of the molecule, preferentially a 5-methylenethiazolidine ring. A total of 39 compounds inhibited YopH in a competitive manner with Ki values <100 μM. The structures and kinetic data for a representative set of 20 analogs are given in Table 3.

**Table 3: Kinetic data of 20 representative analogs**

| Rank # | ID # | Structure | Ki(c) [μM] | Ki(u) [μM] |
|---|---|---|---|---|
| 1 | 5557271 | 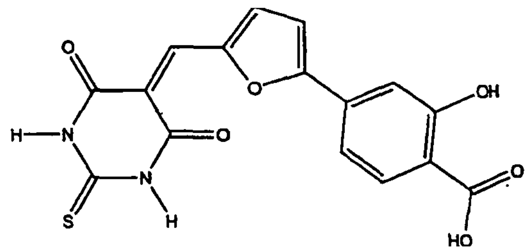 | **0.330** ± | |
| 2 | 5670901 | 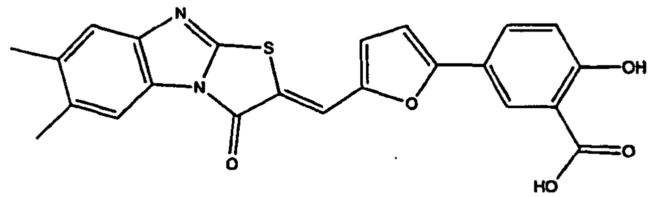 | **0.922** | 8.3 |
| 3 | 5680029 | 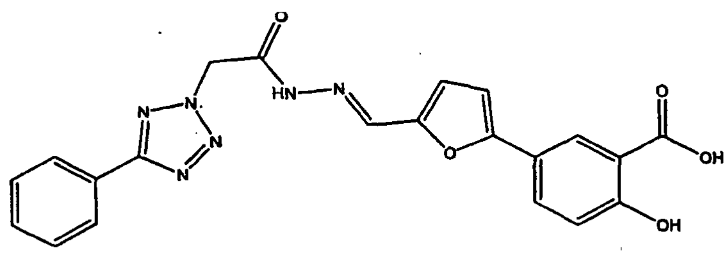 | **2.80** | 8.9 |
| 4 | 5378650 | 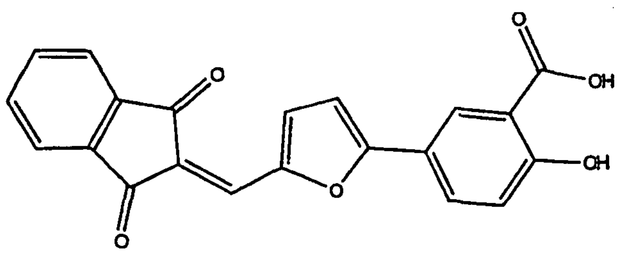 | **3.20** | |
| 5 | 5381181 | 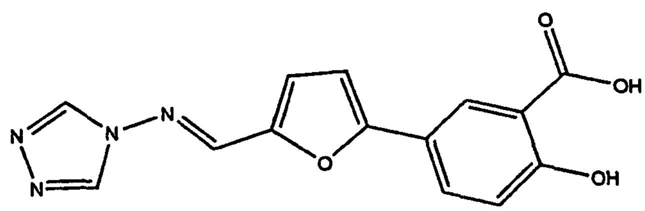 | **3.73** | |
| 6 | 5377894 | 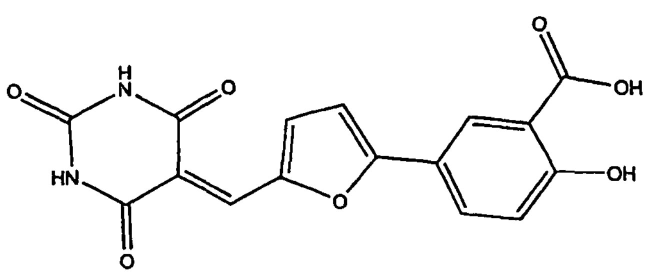 | **4.13** | |

(continued)

| Rank # | ID # | Structure | Ki(c) [μM] | Ki(u) [μM] |
|---|---|---|---|---|
| 9 | 6701338 | 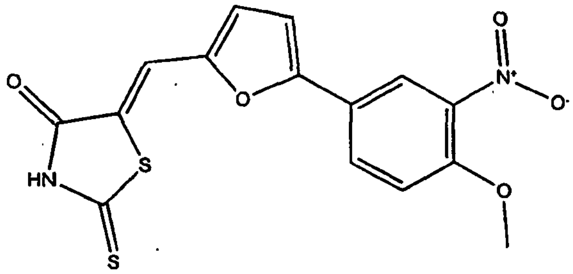 | **6.53** | 9.8 |
| 11 | 8164075 | 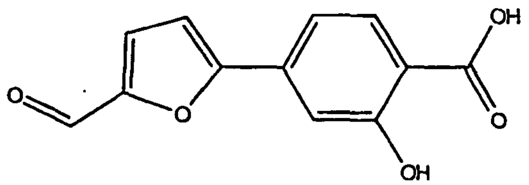 | **7.89** | 61.2 |
| 13 | 5675844 | 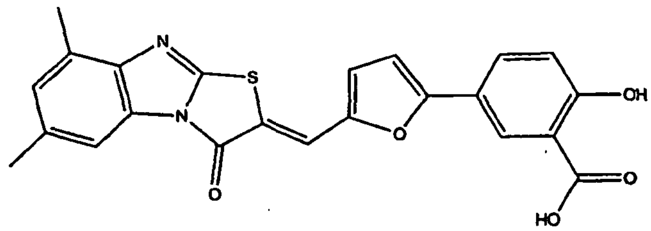 | **8.82** | 10.9 |
| 16 | 6338530 | 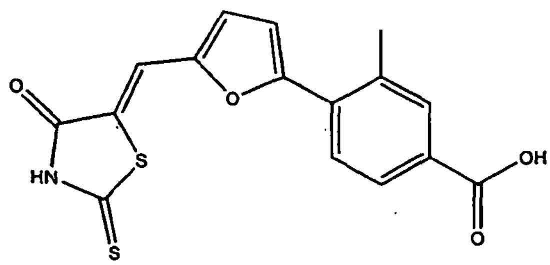 | **11.4** | 84.2 |
| 20 | 6431206 | 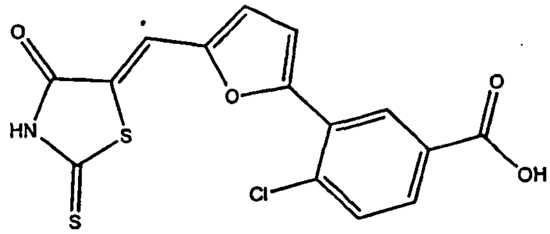 | **16.2** | |
| 21 | 5740279 | 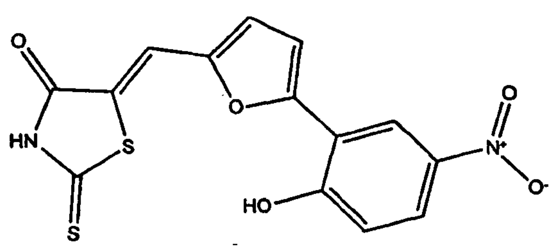 | **17.1** | 16.5 |

(continued)

| Rank # | ID # | Structure | Ki(c) [μM] | Ki(u) [μM] |
|---|---|---|---|---|
| 22 | 5680282 | 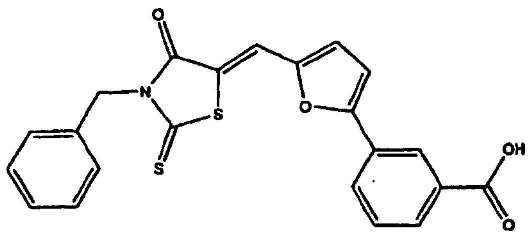 | **17.7** | 42.8 |
| 25 | 5377688 | 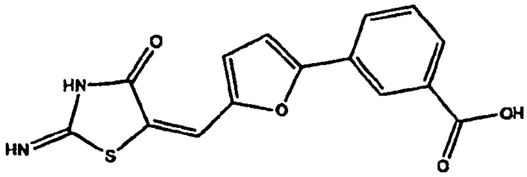 | **19.2** | |
| 27 | 5660015 | 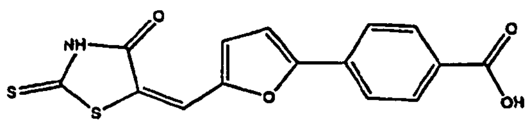 | **19.7** | |
| 30 | 5667408 | 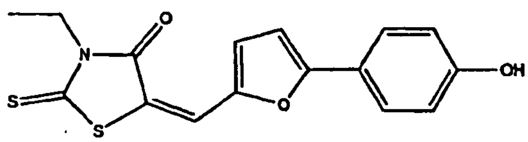 | **38.6** | 55.7 |
| 50 | 5376305 (illustrative) | 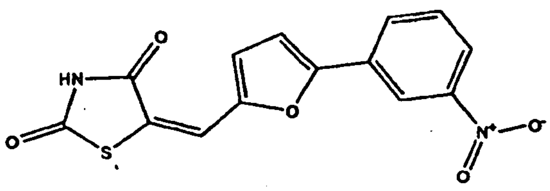 | | 50.0 |
| 54 | 6145062 (illustrative) | 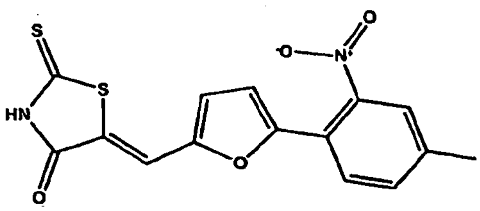 | | 94.1 |
| 60 | 572885 (illustrative) | 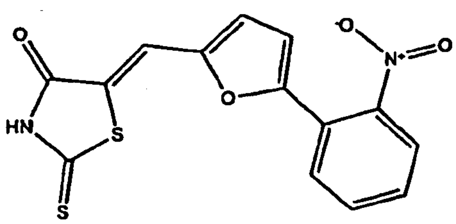 | - | - |
| 61 | 5377816 (illustrative) | 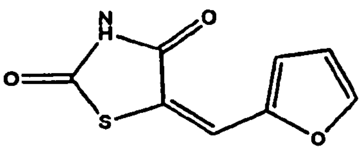 | - | - |

Significantly, 12 of the 14 best compounds (competitive inhibition with Ki values <10 μM) contained salicylic acid. Elimination of the salicylic acid moiety (compound 56377816) led to a complete loss of YopH inhibition. Compared

to analogs with a nitro group instead of a carboxylic group as substituent on the phenyl ring, salicylates were more competitive inhibitors and had lower Ki values. This probably reflects the more phosphate-like hydrogen-bond donor/acceptor properties of the carboxylic group. However, salicylic acid alone was a poor inhibitor (Ki = 882 μM) compared to the most potent inhibitor, 4-(5-((tetrahydro-4,6-dioxo-2-thioxopyrimidin-5(6H)-ylidene)methyl)furan-2-yl)-salicylic acid (compound 5557271), which has a 2667-fold lower inhibitory constant (Ki = 0.33 μM). Thus, the furanyl ring confers both efficacy and specificity to the inhibitors.

The 12.5-fold higher Ki value of the structurally similar compound 5377894 (Ki = 4.1 μM), supports the notion of conformation-specific binding. In the, compound 5377894, the methylidene double bond are changed to cis, and the positions of the carboxylic and the hydroxyl groups are switched. An almost ten-fold difference in inhibitory efficacy between compounds 5670901 (Ki = 0.92 μM) and 5675844 (Ki = 8.8 μM) was observed. These compounds only differ by the location of a single methyl group. This suggests some steric constraints for the putative binding site.

The structure-activity relationship analysis resulted in the identification of three compounds (, which inhibited YopH with lower Ki values than the first furanyl-salicylate hit, compound 5842540. A comparison of the Lineweaver-Burk plots for these four is shown in Fig. 1.

### Example 4

### Virtual Docking Studies

To provide further insights into specific interactions of our inhibitors with the enzyme, we decided to do some molecular modeling with the known structure of the C-terminus of YopH. Flexible-ligand docking was performed with the best inhibitors listed in Table 4 utilizing the X-ray coordinates of the catalytic domain of YopH. Stuckey, et al., Nature 370: 571-575,1994.

**Table 4: Interactions of inhibitors with YopH**

| ID | Structure | $K_D$ (μM) | E FlexX (kJ/mol) | H-Bond |
|---|---|---|---|---|
| **5557271** | 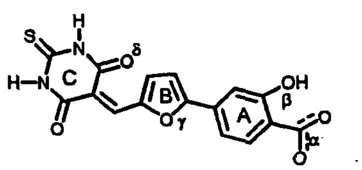 | 0.33 | -46.5 | Gly 408H (α)<br>Arg 409H (α)<br>Val 407H (α)<br>Ser 403 Hγ (α)<br>Thr 410 H (α)<br>Arg 409Hη (β)<br>Ala 405H (β)<br>Arg 404H (β)<br>Gln 357Hε (γ)<br>Gln 446 Hε (γ)<br>Gln 446 He (δ)<br>Gln 357Hε (8) |
| **5670901** | 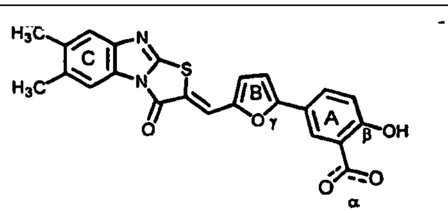 | 0.54 | -35.8 | Arg 409 Hε (α)<br>Arg 409 H (α)<br>Ala 405 H (β)<br>Arg 409 Hγ (β)<br>Val 407 Hγ (β)<br>Ser 403 Hγ (β)<br>Gln 357 Hε (γ)<br>Arg 404 Hη (γ) |
| **5680029** | 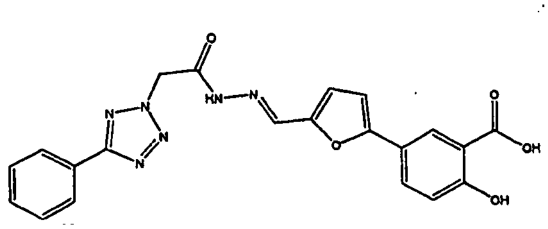 | 1.04 | -44.3 | Gln 450 Hε (α)<br>Arg 409 Hη (α)<br>Gln 357 Hε (γ)<br>Gln 446 Hε (γ)<br>Arg 205 Hη (δ)<br>Gln 357 Hε (ε) |

(continued)

| ID | Structure | $K_D$ (μM) | E FlexX (kJ/mol) | H-Bond |
|---|---|---|---|---|
| **5842540** | 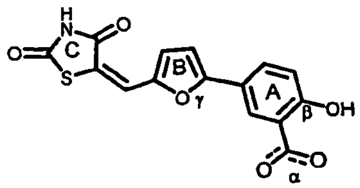 | 1.34 | -34.4 | Val 407 H (α)<br>Arg 409 He (α)<br>Arg 409 H (α)<br>Gly 406 H (α)<br>Ser 403 Hγ (α)<br>Ala 405H (β)<br>Arg 404H (β)<br>Arg 409Hη (β)<br>Gln 357 He (γ)<br>Gln 446 He (γ) |

In all cases, there was a high degree of convergence for the salicylic-furanyl moiety, which occupied the deep hydrophilic phosphate binding cavity (catalytic pocket) on the surface of YopH (Fig. 2). The salicylic group (Table 4, Ring A) appears to be involved in a complex network of hydrogen bonding interactions (Fig. 2A,C,E,G, Table 4) that correlate very well with the lower inhibition levels observed in analogues of compounds that lack either the carboxylic and/or the hydroxyl group in Ring A (Table 3). In addition, the oxygen atom of the furan ring (Ring B) is also invariably involved in hydrogen bonding interactions with the side-chains of Gln357 or Arg404 (Table 4, Fig. 2A,C,E,G). From these docking studies, it is likely that most of the binding energy of the four inhibitors resides in the above mentioned interactions. In fact, docking studies performed with a virtual compound containing only Rings A and B gave similar binding energies whereas the in silico elimination of the carboxylic and/or the hydroxyl groups in Ring A produced compounds that fail to dock in the catalytic pocket of the protein. For example, the binding energy of compound 5557271 dropped from -46 KJ/mol (Table 4) to - 21 KJ/mol after removal of the carboxylic and hydroxyl groups. Further evidence of the key role played by the Rings A and B through experimental verification that the compound 8164075, despite lacking Ring C, is still capable of inhibiting YopH in the low micromolar range (Table 3). The position of Ring B with respect to Ring A also seems to play an important role in defining the binding properties of the compounds. In fact, it appears that when the carboxylic acid in Ring A is in para position with respect to Ring B, a denser network of hydrogen binding interactions is possible within the YopH catalytic pocket (Table 4, Fig. 2A,C,E,G). In all four inhibitors, the positioning of ring C was less defined among the 20 solutions generated with FlexX, again correlating with the high variability of tolerated substitutions at this position. However, a few important conclusions can be made. In the highest scoring solutions for compound 5557271, Ring C is involved in an additional hydrogen bonding interaction with the side chains of Gln357 and Gln446 whereas similar interactions occur with compound 5680029 and Arg205, that can confer further affinity for YopH. The methyl groups in Ring C of compound 5670901 make favorable steric contacts with YopH, in very close proximity to an additional grove (termed 'P2' in Fig. 3) on the surface of the protein. Based on the latter model, one can also predict that even small substitutions in Ring C at the improper position could result in unfavorable steric hindrance and decrease the binding affinity, as observed for compound 5675844 (Table 3).

**Example 5**

**Selectivity For YopH**

To evaluate whether the best 4 inhibitors were sufficiently selective for YopH compared to PTPases from other sources, the catalytic pocket and its surroundings were inspected from two other PTPases, namely PTP1B and VHR. Barford, et al., Science 263: 1397-1404, 1994; Yuvaniyama et al., Science 272: 1328-1331, 1996. The crystal structures of PTPases, PTP1B and VHR, have been solved. Although these PTPases have very similar catalytic cores, they differ dramatically in surface topology around the catalytic pocket (Fig. 3). None of the four best furanyl-salicylate-compounds bound well to PTP1B or VHR in silico. (data not shown). Direct measurements of $IC_{50}$ values for a set of PTPases of the four inhibitors showed that they inhibited YopH at lower concentrations than any of the tested enzymes (Table 5). The enzymes in Table 5 are representative of all subfamilies of PTPases (except CDC25, which did not dephosphorylate the pNPP substrate). The furanyl-salicylates compounds were selective inhibitors for YopH PTPase.

**Table 5: Selectivity of inhibitors**

| | 5557271 **IC50 [μM]** | 5670901 **IC50 [μM]** | 5842540 **IC50 [μM]** | 5680029 **IC50 [μM]** |
|---|---|---|---|---|
| YopH | **1.4** | **2.0** | **3.9** | **5.1** |
| PTP 1B | **9.7** | **7.0** | **12.0** | **14.3** |
| TCPTP | **11.0** | **11.0** | **30.2** | **22.7** |
| LAR | 10.9 | 7.7 | 12.2 | 35.8 |
| CD45 | **1.7** | **2.5** | **5.7** | **15.1** |
| VHR | **34.7** | **21.2** | **27.5** | **31.6** |
| VH1 | >250 | >250 | >250 | >250 |
| VHX | >250 | >250 | 38.5 | 34.9 |
| HePTP | **32.4** | **32.6** | **34.5** | **47.9** |
| LMPTPB | >250 | >250 | 215 | >250 |

**Example 6**

**Restoration of Tyrosine Phosphorylation and TCR Signaling in YopH Expressing Cells**

The four inhibitors are tested in live cell assays, in which membrane-permeable YopH is added to normal human T lymphocytes. The PTPase causes a dramatic reduction in tyrosine phosphorylation and T cell antigen receptor-mediated signal transduction and activation to produce interleukin-2. Alonso, et al., J. Biol. Chem. 279: 4922-4928, 2004. The four YopH inhibitors will be added at different concentrations and will be assessed for their ability to reverse the dephosphorylation of cellular proteins.

**Example 7**

**Synthesis of inhibitors of *Yersinia* spp. protein tyrosine phosphatase**

*2-(4-Oxo-2-thioxo-thiazolidin-3-yl)-N-phenethyl-acetamide* (*1*) (preparative)

*N*-Cyclohexylcarbodiimide-*N'*-propylmethyl polystyrene (PS-CDI resin, 819 mg, 1 mmol) was added to a dry round bottomed flask Rhodanine acetic acid (143 mg, 0.75 mmol) was added as a solution in $CH_2Cl_2$ (4 ml) and the reaction mixture was stirred at room temperature. After 5 minutes, phenyl ethyl amine (61 mg, 0.5 mmol) in 4 ml of $CH_2Cl_2$ was added and the suspension stirred at room temperature overnight. The reaction mixture was filtered under vacuum and the resin was washed twice with $CH_2Cl_2$. The organic phase was dried over $Na_2SO_4$ and the solvent evaporated to give a whitish solid that was used for the following step with no further purification.

*2-Hydroxy-4-{5-(4-oxo-3-(phenethylcarbamoyl-methyl)-2-thioxo-thiazolidin-5 ylidenemethyl]-furan-2-yl)-benzoic acid* (*2*) (illustrative)

Compound 1 (59 mg, 0.20 mmol was dissolved in DMF (1 ml and to the mixture was added 4-(5-formyl-furan-2-yl)-2-hydroxy-benzoic acid (50 mg, 0.22 mmol). The solution was reacted in a microwave following four cycles of 1 minute heating (140˚C, 1000 W) and 3 minutes of cooling down (25 ˚C). After adding water to the reaction mixture the final compound precipitated. The solid was recovered by filtration and dried to give 57 mg (56% yield) of a yellow powder. $^{13}C$ NMR (*d*-DMSO, 75 MHz): 194.6, 172.1, 167.0, 165.0, 162.1, 157.0, 150.8, 140.0, 135.3, 132.4, 132.0, 129.5, 129.3, 129.1, 126.8, 123.7, 120.5, 119.2, 115.9, 113.9, 113.6, 112.8, 46.9, 41.2, 35.6. MALDI-MS *m/z* (%): 531 (5, $M^+$ + Na), 361 (8), 330 (17), 273 (100).

*2-Hydroxy-5-{5-[4-oxo-3-(phenethylcarbamoyl-methyl)-2-thioxo-thiazolidin-5-ylidenemethyl]-furan-2-yl}-benzoic acid (3)*

Compound **1** (61 mg, 0.21 mmol) was dissolved in DMF (1 ml) and to the mixture was added 5-(5-formyl-furan-2-yl)-2-hydroxy-benzoic acid (54 mg, 0.23 mmol). The solution was reacted in a microwave following four cycles of 1 minute heating (140 °C,1000 W) and 3 minutes of cooling down (25 °C). After adding water to the reaction mixture the final compound precipitated. The solid was recovered by filtration and dried to give 57 mg (58% yield)of an orange powder.$^{13}$CNMR (*d*-DMSO, 75 MHz): 194.6, 172.0, 167.0, 165.1, 162.5, 158.3, 149.5, 140.0, 132.3, 129.4, 129.1, 127.1, 126.9, 124.4, 120.8, 119.3, 119.2, 118.5, 114.7, 109.9, 46.8, 41.2, 35.7. MALDI-MS m/z (%): 532 (54, $M^+$ + Na), 477 (65), 387 (32), 361 (8), 330 (17), 282 (35), 273 (100).

**Example 8**

**Materials**

*p*-Nitrophenyl phosphate (*p*NPP) was purchased from Sigma (St. Louis, MO). BIOMOL GREEN™ Reagent was purchased from BIOMOL® Research Laboratories, Inc. (Plymouth Meeting, PA). All other chemicals and reagents were of the highest grade available commercially. Anti-phosphotyrosine mAb 4G10 and recombinant PTPases were from Upstate Biotechnology Inc. (Lake Saranac, NY) and mAb PY20 from B. D. Biosciences (San Diego, CA).

**Plasmids and Protein Purification**

The eukaryotic and prokaryotic expression plasmids for YopH were as described. YopH was expressed and purified as described previously. Alonso, et al., J. Biol. Chem. 279: 4922-4928, 2004. The PTPases VHX, VHR, VH1, TCPTP, LMPTP, and HePTP were expressed in E. *coli* and purified as described previously. Alonso, et al., J Biol Chem 277: 5524-5528, 2002; Alonso, et al., Nat Immunol 4, 44-48, 2003; Kholod, Biotechniques 31, 322-328, 2001; Saxena, et al., Nature Cell Biol. 1: 305-311, 1999; Saxena, et al., J. Biol. Chem. 273: 15340-15344, 1998.

**Chemical library Screening for YopH Inhibitors**

A subset of 10,000 compounds from the DIVERSet™ library of 50,000 drug-like molecules (ChemBridge, Inc.) were screened in a 96-well format *in vitro* assay. Each reaction contained 50 nM YopH, 1 mM *p*NPP and 0.03 mg/ml compound in 0.1 M Bis-Tris pH 6.0 reaction buffer. The final volume amounted to 50 μl an contained 2 % DMSO. The reaction was initiated by addition of *p*NPP after a preincubation of the enzyme with the compounds for 10 min at room temperature. After 7 min the reaction was quenched by addition of 100 μl BIOMOL GREEN™ Reagent and the *p*NPP hydrolysis was determined by measuring the absorbance of the complexed free phosphate at 620 nm. The nonenzymatic hydrolysis of the substrate was corrected by measuring the control without addition of enzyme. To quantitate the inhibitory efficacy of the library compounds, we determined the ratio of inhibition in comparison to 200 μM orthovanadate, a PTPase inhibitor. Every compound with an ratio >1 was considered as a hit.

**Ki Determination**

The YopH PTPase-catalyzed hydrolysis of *p*NPP in the presence of inhibitors was assayed at 30 °C in 0.1 M Bis-Tris, pH 6.0 assay buffer containing 5 % DMSO. The enzyme was preincubated with various fixed concentrations of inhibitors for 10 min. The reaction was initiated by addition of various concentrations of *p*NPP (ranging from 0.2 to 10 *Km*) to the reaction mixtures to a final volume of 100 μl. The nonenzymatic hydrolysis of the substrate was corrected by measuring the control without addition of enzyme. The amount of product p-nitrophenol was determined from the absorbance at 405 nm detected by a PowerWaveX340 microplate spectrophotometer (Bio-Tek Instruments, Inc.) using a molar extinction coefficient of 18,000 M-icm-i. The inhibition constant and inhibition pattern were evaluated by fitting the data to the Michaelis-Menten equations for either competitive [1], uncompetitive [2] or mixed [3] inhibition, using nonlinear regression and the program GraphPad Prism® (version 4.0).

$$v_0 = V_{max}[S]/(K_{mapp}+[S]) \text{ with } K_{mapp} = K_m(1+[I]/K_i) \qquad [1]$$

$$v_0 = V_{max}[S]/(K_m+(1+[I]/K_i)[S]) \text{ with } K_{mapp} = K_m/(1+[I]/K_i) \qquad [2]$$

$$v_0 = V_{max}[S]/((1+[I]/K_{ic})K_m+(1+[I]/K_{iu})[S]) \text{ with } K_{mapp} = K_m(1+[I]/K_{ic})/(1+[I]/K_{iu}) \quad [3]$$

In the case of the mixed inhibition model $K_{ic}$ is the inhibition constant for the competitive participation and $K_{iu}$ the inhibition constant for the uncompetitive participation. For a comparison of the fitting results the second-order Akaike's Information Criterion (AICc) was calculated with equation [4], where N is the number of data points, SS the absolute sum of squares and K the number of parameters fit by nonlinear regression plus 1.

$$AIC_c = N\ln(SS/N)+2K+(2K(K+1))/(N-K-1) \quad [4]$$

The probability to have chosen the right model can be computed by equation [5], where $\Delta$ is the difference between AICc scores.

$$\text{probability} = \exp(-0.5\Delta)/(1+\exp(-0.5\Delta)) \quad [5]$$

### IC50 Measurements

The PTPase-catalyzed hydrolysis of *p*NPP in the presence of inhibitor was assayed at 30 °C in a 100 μl reaction system in the same assay buffer described above. At various concentrations of the compound, the initial rate at fixed *p*NPP concentrations (equal to the corresponding *K*m values for each PTPase) was measured by continuously following the production of *p*-nitrophenol as described above; The IC50 value was determined by plotting the relative *p*NPP activity versus inhibitor concentration and fitting to equation [6] using GraphPad Prism®.

$$V_i/V_0 = IC_{50}/(IC_{50} + [I]) \quad [6]$$

In this case, Vi is the reaction velocity when the inhibitor concentration is [I], Vo is the reaction velocity with no inhibitor, and IC50= Ki+ Ki[S]/Km. Therefore, when the substrate concentration [S] is equal to Km, IC50= 2Ki.

### Molecular Modeling

Molecular modeling studies were conducted on several R12000 SGI Octane workstations with the software package Sybyl version 6.9 (TRIPOS). Energy-minimized molecular models of the compounds were generated by the Sybyl/MAXIMIN2 routine. Flexible ligand docking calculations were performed with FlexX as implemented in Sybyl. For each compound, 20 solutions were generated and raked ordered via FlexX score and CSCORE. In all cases, there was a high degree of convergence for the salicylic acid - furanyl moiety and more variability in the position of the remaining of the molecules. The coordinates of three-dimensional structure of catalytic domain of YopH (PDB code 1YTS; Stuckey, et al., Nature 370: 571-575,1994; and 1QZ0 (Stuckey, et al., Nature 370: 571-575, 1994) were used in the docking studies and the binding pocket was defined as composed by amino acid residues: Arg205, Arg228, Phe229, Ile232, Asn245, Ala258, Cys259, Gln260, Tyr261, Val284, Leu285, Ala286, Ser287, Glu290, Ile291, Phe296, Met298, Val351, Trp354, Pro355, Asp356, Gln357. Thr358, Ala359, Val360, Ile401 , His402, Ser403 (Cys403 in wild-type YopH), Arg404, Ala405, Gly406, Val407, Gly408, Arg409, Thr410, Ala411, Gln412, Leu413, Ile414, Arg440, Asn441, Ile443, Met444, Val445, Gln446, Lys447, Gln450. Molecular surfaces were generated with MOLCAD as implemented in Sybyl. Comparisons with other PTPases were made by using the X-ray coordinates of PTP1B (PDC code 1PTY) and VHR (PDB code 1VHR).

### Cells and cell treatments

Normal T lymphocytes were isolated from venous blood of healthy volunteers by Ficoll gradient centrifugation. Monocytes/macrophages were eliminated by adherence to plastic for 1 h at 37°C. Jurkat T leukemia cells were kept at logarithmic growth in RPMI 1640 medium supplemented with 10% fetal calf serum, 2 mM L-glutamine, 1mM sodium pyruvate, nonessential amino acids and 100 units/ml each of penicillin G and streptomycin. For TCR and CD28 induced

tyrosine phosphorylation responses, normal T lymphocytes were incubated in ice for 15 min with 10 μg/ml OKT3 and anti-CD28 mAbs, washed, and incubated with a crosslinking sheep anti-mouse Ig for 15 min, washed and transferred to 37˚C for 5 min. Cells were pelleted and lysed in 20 mM Tris-HCl, pH 7.5, 150 mM NaCl, 5 mM EDTA containing 1% NP-40, 1 mM $Na_3VO_4$, 10 μg/ml aprotinin and leupeptin, 100 μg/ml soybean trypsin inhibitor and 1 mM phenylmethylsulphonyl fluoride and clarified by centrifugation at 15,000 rpm for 20 min. Lysate was mixed with an equal volume of twice concentrated SDS sample buffer, boiled for 1 min, and resolved by SDS PAGE.

**SDS PAGE and immunoblotting**

These procedures were done as before. Alonso, et al., J. Biol. Chem. 279: 4922-4928, 2004.

**Interleukin-2 secretion assay**

5 x $10^6$ human T lymphocytes were treated with 6 μM ANT-YopH for 5 h at 37˚C in RPMI medium, washed, and stimulated with C305, anti-CD28 mAb, plus a crosslinking anti-mouse Ig for 15h in 250 μl of medium with 10% FCS. 20 μl of the supernatant was used for measurement of the amount of interleukin-2 using an enzyme-linked immunosorbent assay kit from Roche Molecular Biochemicals (Mannheim, Germany), as before[39]. Results are given as pg/ml of secreted interleukin-2/$10^6$ cells.

## Claims

**1.** A *Yersinia* spp. protein tyrosine phosphatase inhibitor selected from the group consisting of:

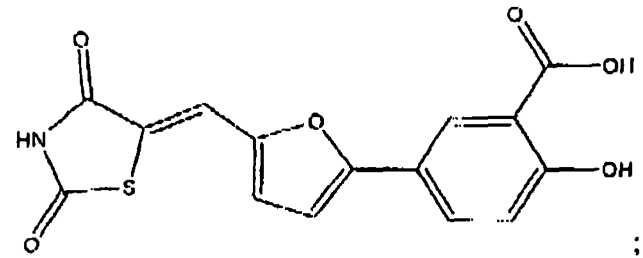

;

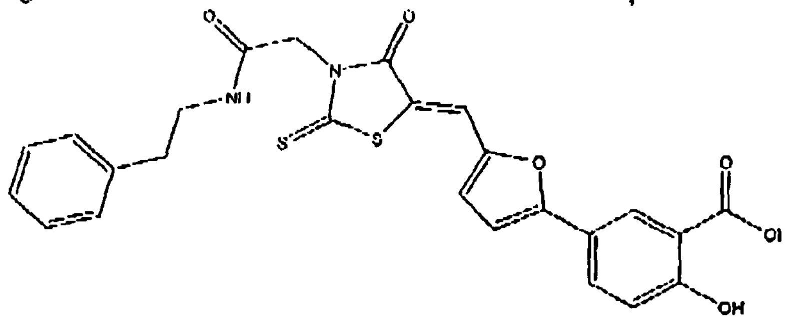

;

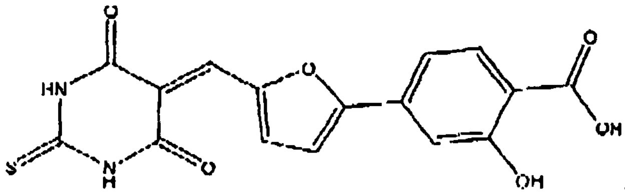

;

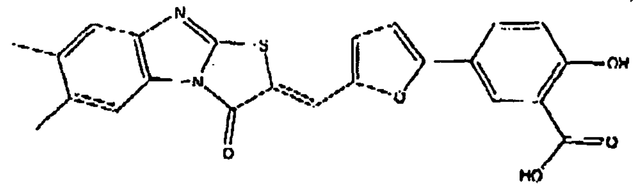

;

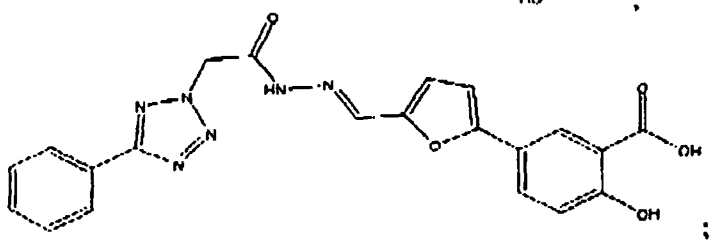

;

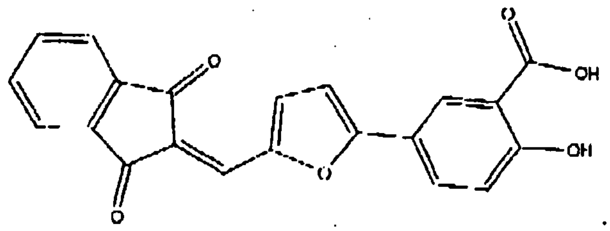

;

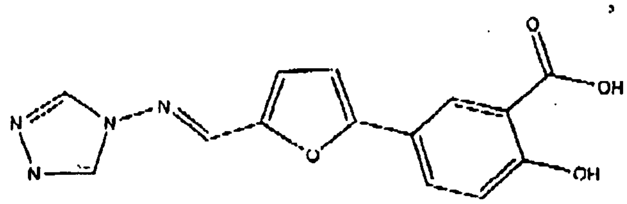

;

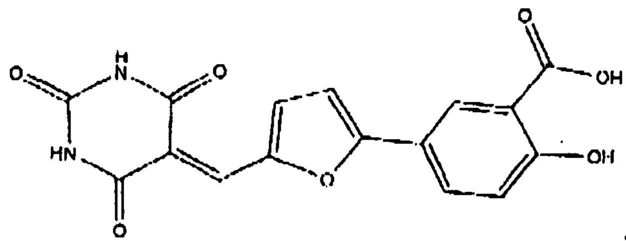

;

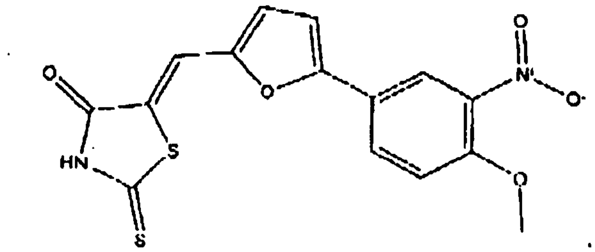

;

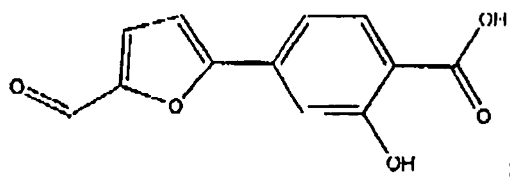

;

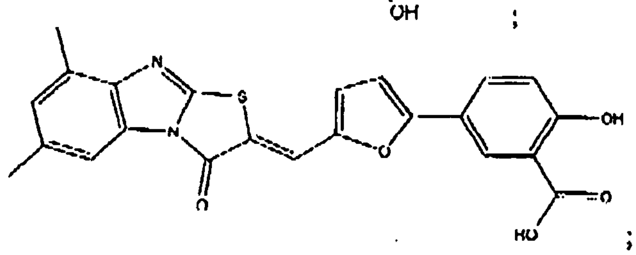

;

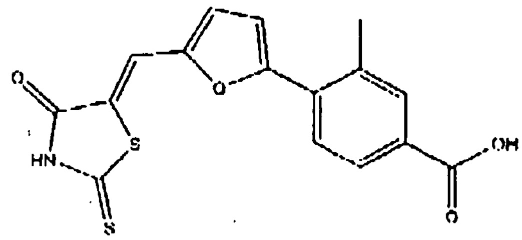

;

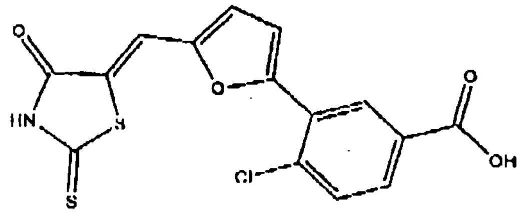

;

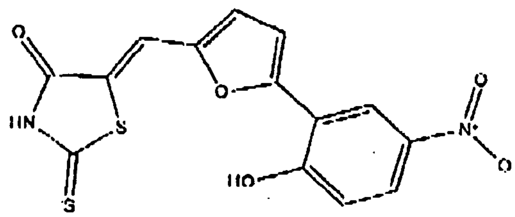

;

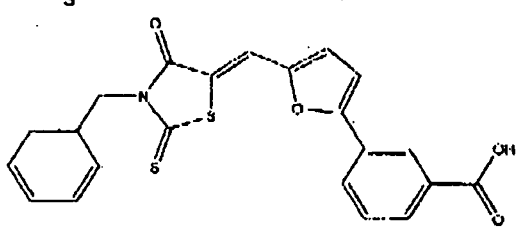

;

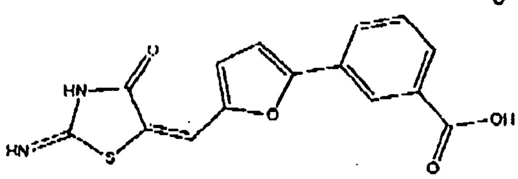

;

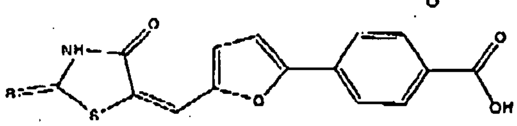

;

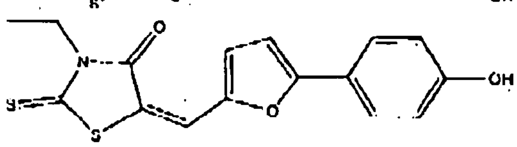

,

and pharmaceutically acceplable salt formes thereof,
for use in treating of a disease, state in a mammal caused by *Yersinia s*pp. infection,
wherein the pharmaceutically acceptable salts are mineral or organic acid salts of basic residues and alkali or organic salts of acidic residues.

**2.** An inhibitor according to claim 1, wherein the protein tyrosine phosphatase inhibitor is selected from:

(i)2-hydroxy-5-{5-[4-oxo-3-(phenethylcarbamoyl-methyl)-2-thioxo-thiazolidin-5-ylidenemethyl]-furan-2-yl}-benzoic acid;
(ii) 5-[5-(6,7-dimethyl-3-oxo-benzo[4,5]imidazo[2,1-b]thiazol-2-ylidenemethyl)-fluran-2-yl]-2-hydroxy-benzoic acid;
(iii) 5-[5-(2,4-dioxo-thiazolidin-5-ylidenemethyl)-furan-2-yl]-2-hydroxy-benzoic acid;
(iv) 4-[5-(4,6-dioxo-2-thioxo-tetrahydro-pyrimidin-5-ylidenemethyl)-furan-2-yl]-2-hydroxy-benzoic acid;

(v) 2-hydroxy-5-(5-{[2-(5-phlenyl-tetrazol-2-yl)-acetyl]-hydrazolomethyl}-furan-2-yl)benzoic acid; and
(vi) pharmaceutically acceptable salt forms thereof, wherein the pharmaceutically acceptable salts are mineral or organic acid salts of basic residues and alkali or organic salts of acidic residues.

## Patentansprüche

**1.** Inhibitor der Proteintyrosinphosphatase aus *Yersinia* spp., ausgewählt aus der Gruppe bestehend aus:

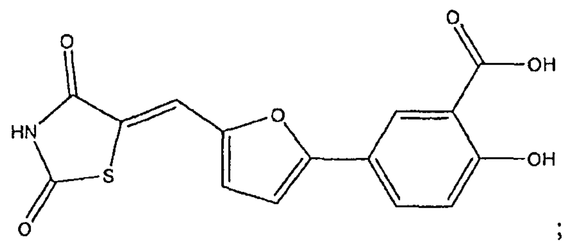
;

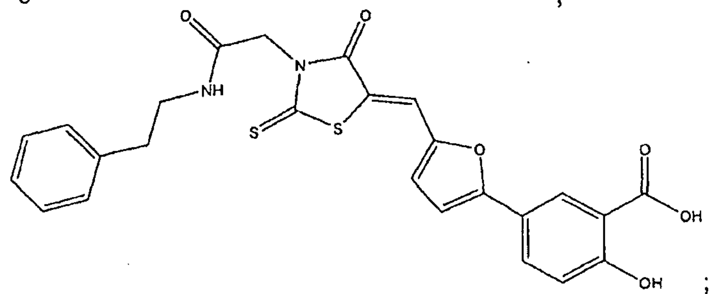
;

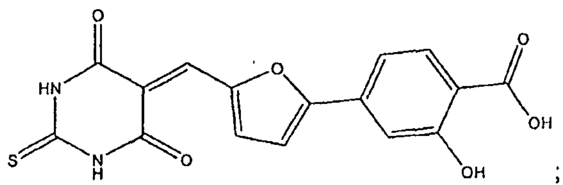
;

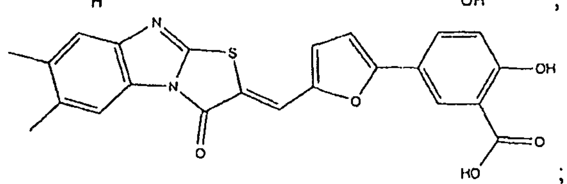
;

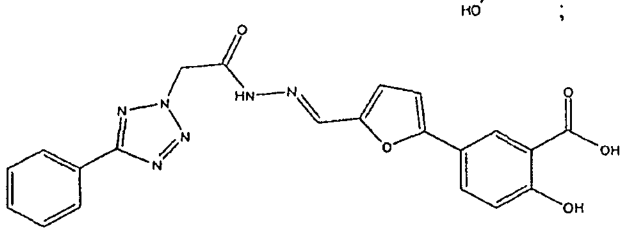
;

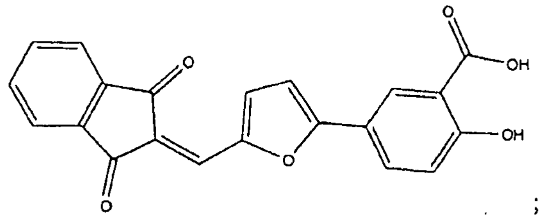

;

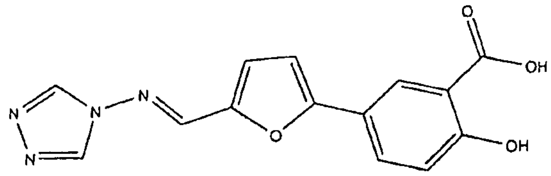

;

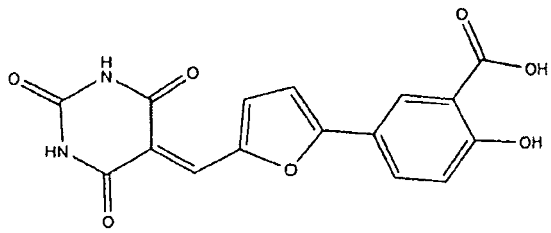

;

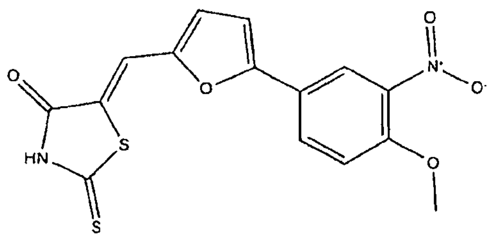

;

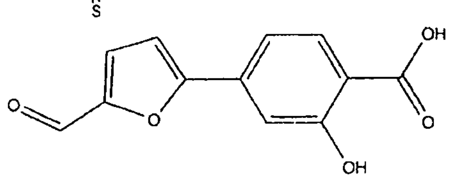

;

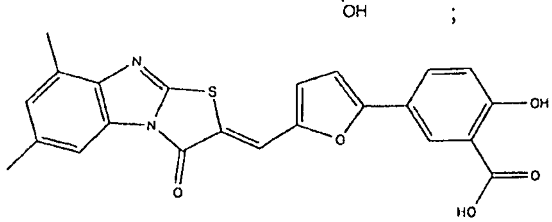

;

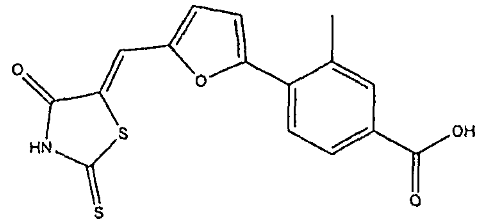
;

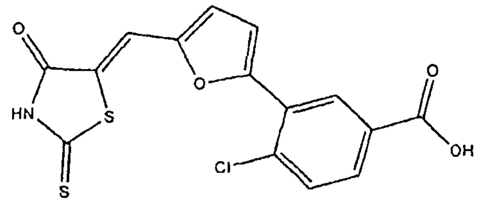
;

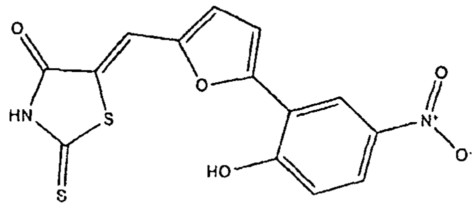
;

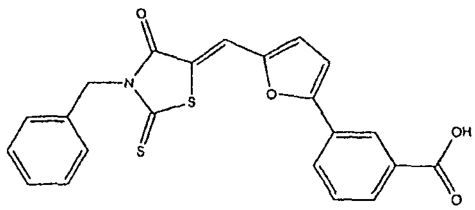
;

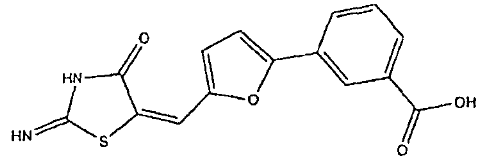
;

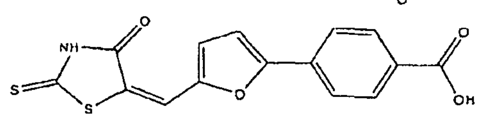
;

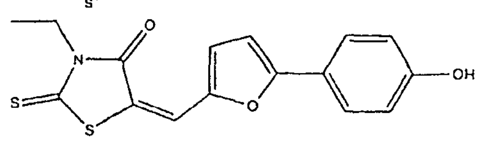
,

und pharmazeutisch akzeptable Salze hiervon,
zur Verwendung in der Behandlung eines Krankheitszustandes in einem Säugetier, der durch eine Infektion durch *Yersinia* spp. verursacht ist,
wobei die pharmazeutisch akzeptablen Salze mineralische oder organische Hydrogensalze von basischen Resten und Alkali oder organische Salze von sauren Resten sind.

**2.** Inhibitor gemäß Anspruch 1, wobei der Proteintyrosinphosphataseinhibitor ausgewählt ist aus:

(i) 2-Hydroxy-5-{5-[4-oxo-3-(phenethylcarbamoyl-methyl)-2-thioxo-thazolidin-5-ylidenmethyl]-furan-2-yl}-benzoesäure;
(ii) 5-[5-6,7-dimethyl-3-oxo-benzo[4,5]imidazol[2,1-b]thiazol-2-ylidenmethyl)-furan-2-yl]-2-hydroxy-benzoesäure;
(iii) 5-[5-(2,4-dioxo-thiazolidin-5-ylidenmethyl)-furan-2-yl]-2-hydroxybenzoesäure;
(iv) 4-[5-(4,6-dioxo-2-thioxo-tetrahydro-pyrimidin-5-ylidenmethyl)-furan-2-yl]-2-hydroxy-benzoesäure;
(v) 2-hydroxy-5-(5-{[2-(5-phenyl-tetrazol-2-yl)-acetyl]-hydrazolomethyl}-furan-2-yl)benzoesäure; und

(vi) pharmazeutisch akzeptable Salze hiervon, wobei die pharmazeutisch akzeptablen Salze mineralische oder organische Hydrogensalze von basischen Resten und Alkali oder organische Salze von sauren Resten sind.

## Revendications

**1.** Inhibiteur de la protéine tyrosine phosphatase de *Yersinia* spp. choisi parmi le groupe consistant en :

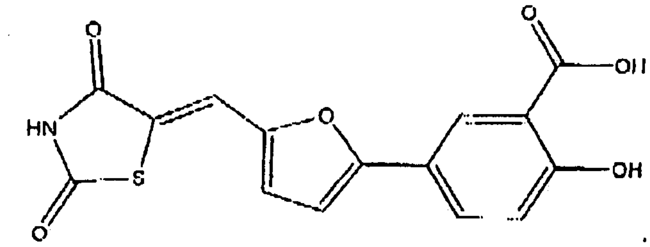

;

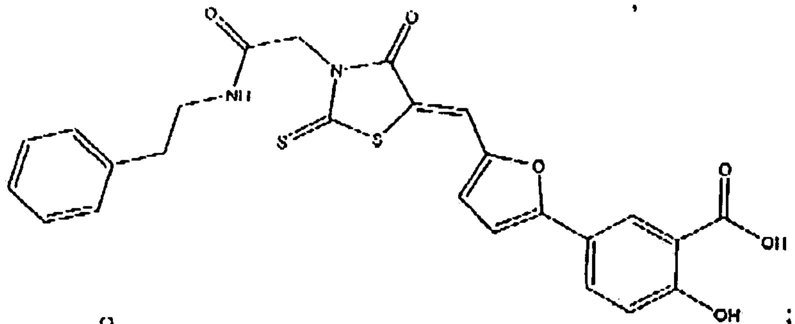

;

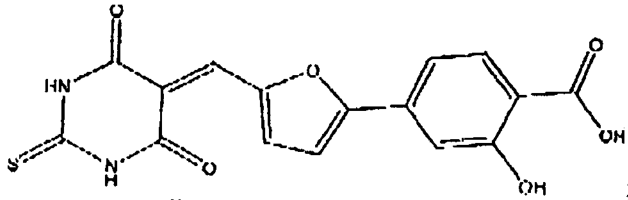

;

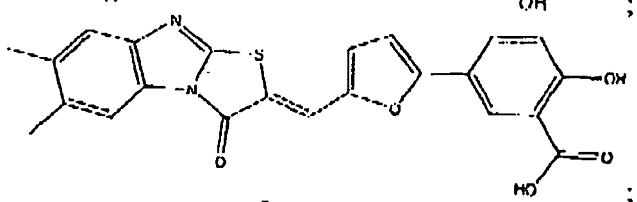

;

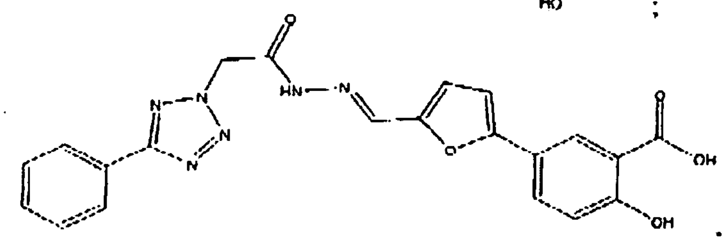

;

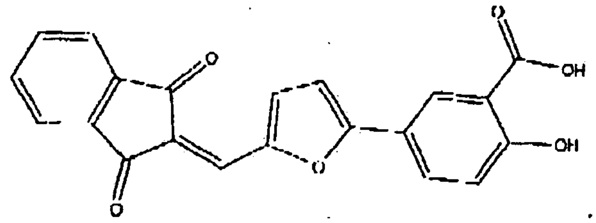

;

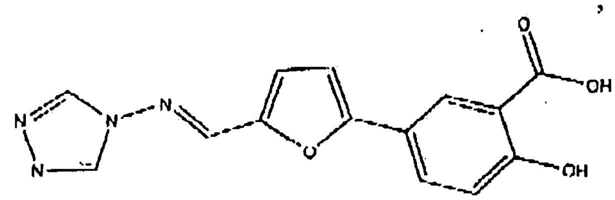

;

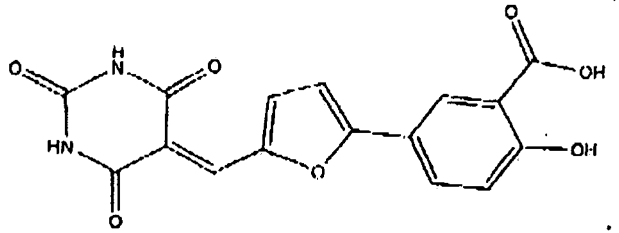

;

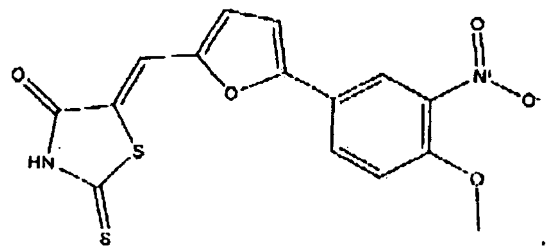

;

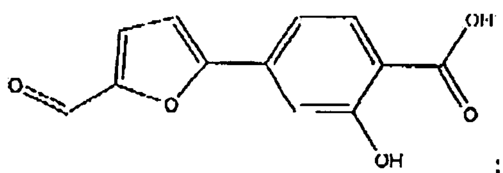

;

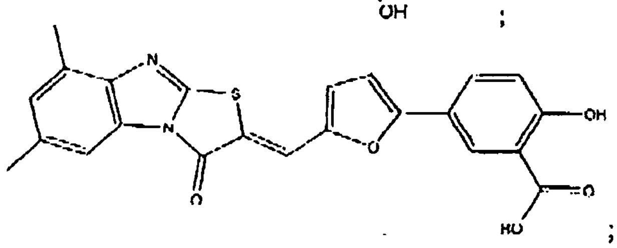

;

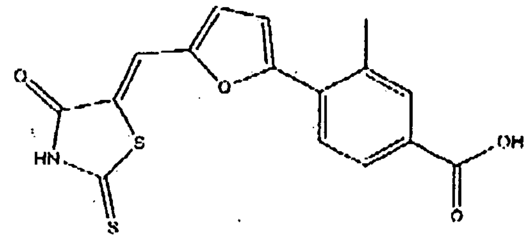
;
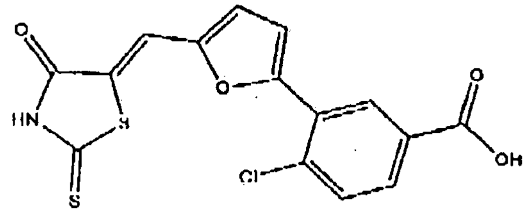
;
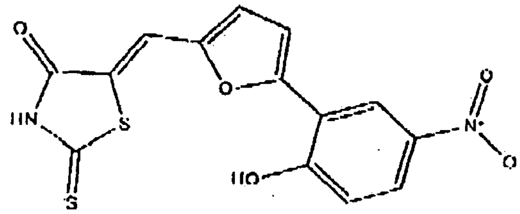
;
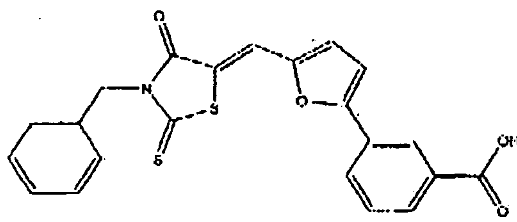
;
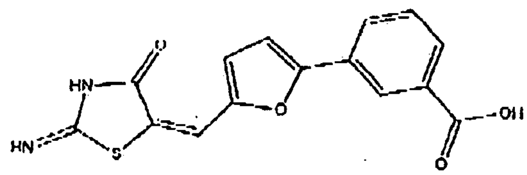
;
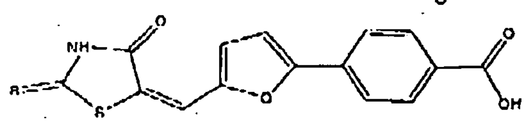
;
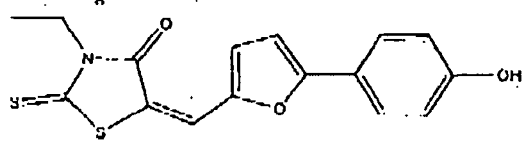
,

et des formes salines pharmaceutiquement acceptables de ces derniers, destiné à être utilisé dans le traitement d'une maladie chez un mammifère due à une infection par *Yersinia* spp.,
où les sels pharmaceutiquement acceptables sont des sels acides minéraux ou organiques de résidus basiques et des sels alcalins ou organiques de résidus acides.

**2.** Inhibiteur selon la revendication 1, où l'inhibiteur de la protéine tyrosine phosphatase est choisi parmi :

(i) l'acide 2-hydroxy-5-{5-[4-oxo-3-(phénéthylcarbamoyl-méthyl)-2-thioxo-thiazolidin -5-ylidèneméthyl]-furan-2-yl}-benzoïque ;
(ii) l'acide 5-[5-(6,7-diméthyl-3-oxo-benzo[4,5[imidazo[2,1-b]thiazol-2-ylidèneméthyl)-furan-2-yl]-2-hydroxy-benzoïque ;
(iii) l'acide 5-[5-(2,4-dioxo-thiazolidin-5-ylidèneméthyl)-furan-2-yl]-2-hydroxy-benzoïque ;
(iv) l'acide 4-[5-(4,6-dioxo-2-thioxo-tétrahydro-pyrimidin-5-ylidèneméthyl)-furan-2-yl]-2-hydroxy-benzoïque ;

(v) l'acide 2-hydroxy-5-(5- {[2-(5-phényl-tétrazol-2-yl)-acétyle]-hydrazolométhyl}- furan-2-yl)-benzoïque ; et
(vi) des formes salines pharmaceutiquement acceptables de ces derniers, lesquels sels pharmaceutiquement acceptables sont des sels acides minéraux ou organiques de résidus basiques et des sels alcalins ou organiques de résidus acides.

**Figure 1: Lineweaver-Burk Plots for the best 4 Inhibitors**

**A.**

**Lineweaver-Burk plot compound 5557271**

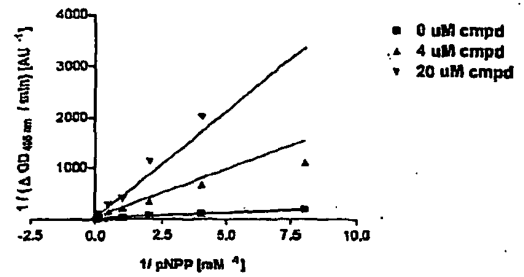


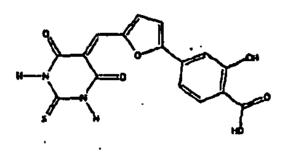

Ki(c) = 0.330 ± 0.0510

**B.**

**Lineweaver-Burk plot compound 5670901**

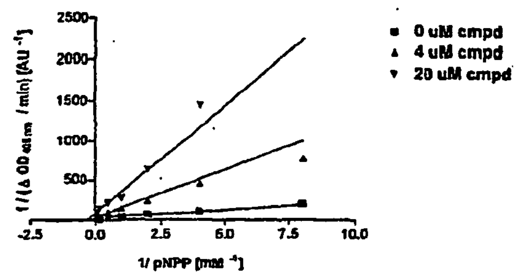


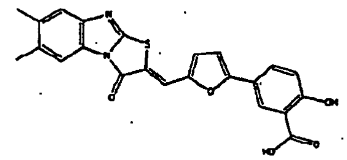

Ki(c) = 0.922 ± 0.0892

**C.**

**Lineweaver-Burk plot compound 5680029**

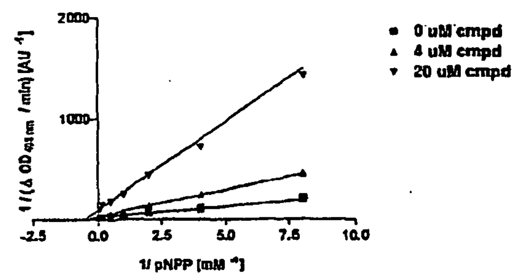


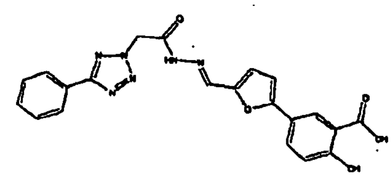

Ki(c) = 2.80 ± 0.395

**D.**

**Lineweaver-Burk Plot for compound 5842540**

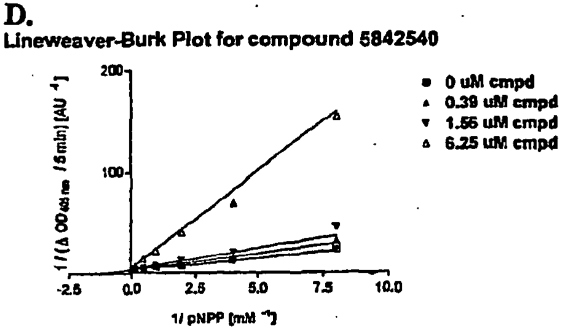


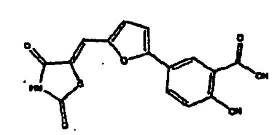

Ki(c) = 1.13 ± 0.198

**Figure 1**

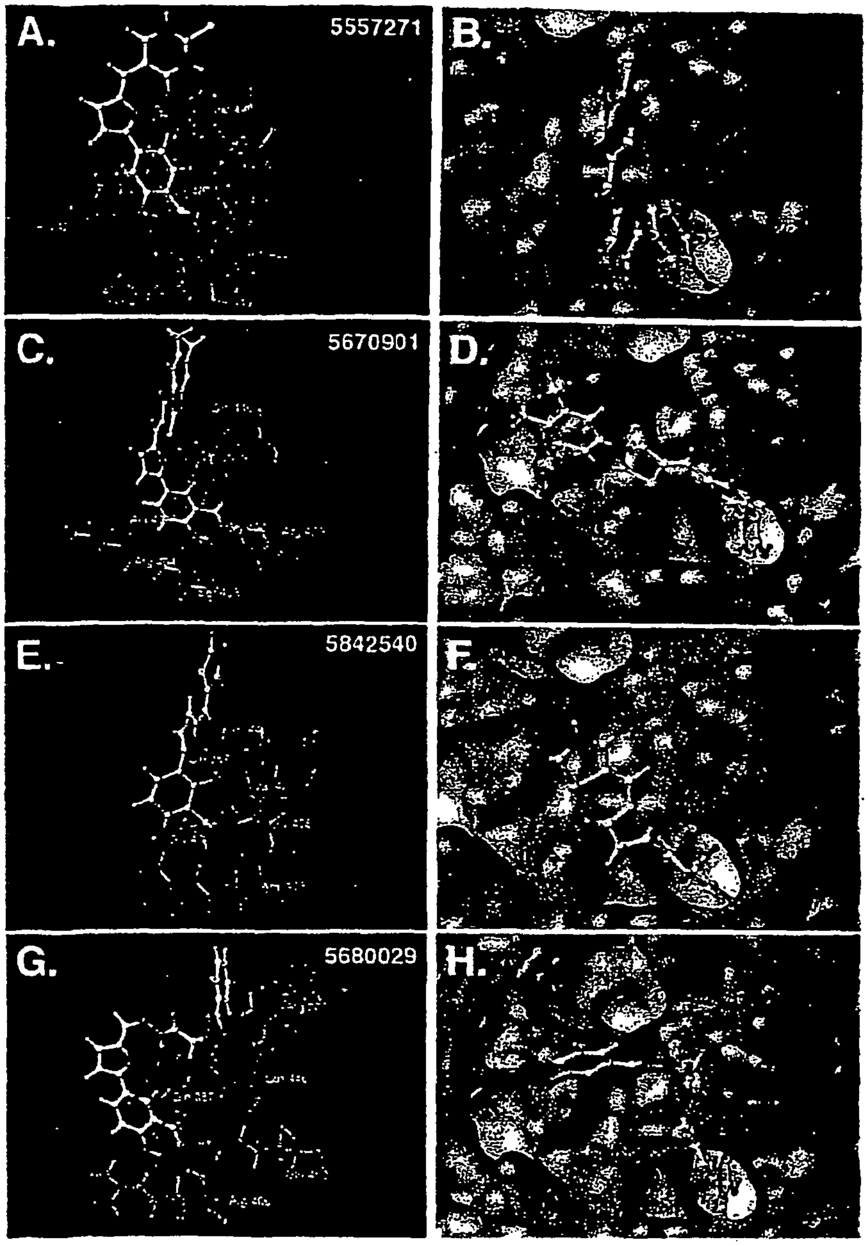


Figure 2

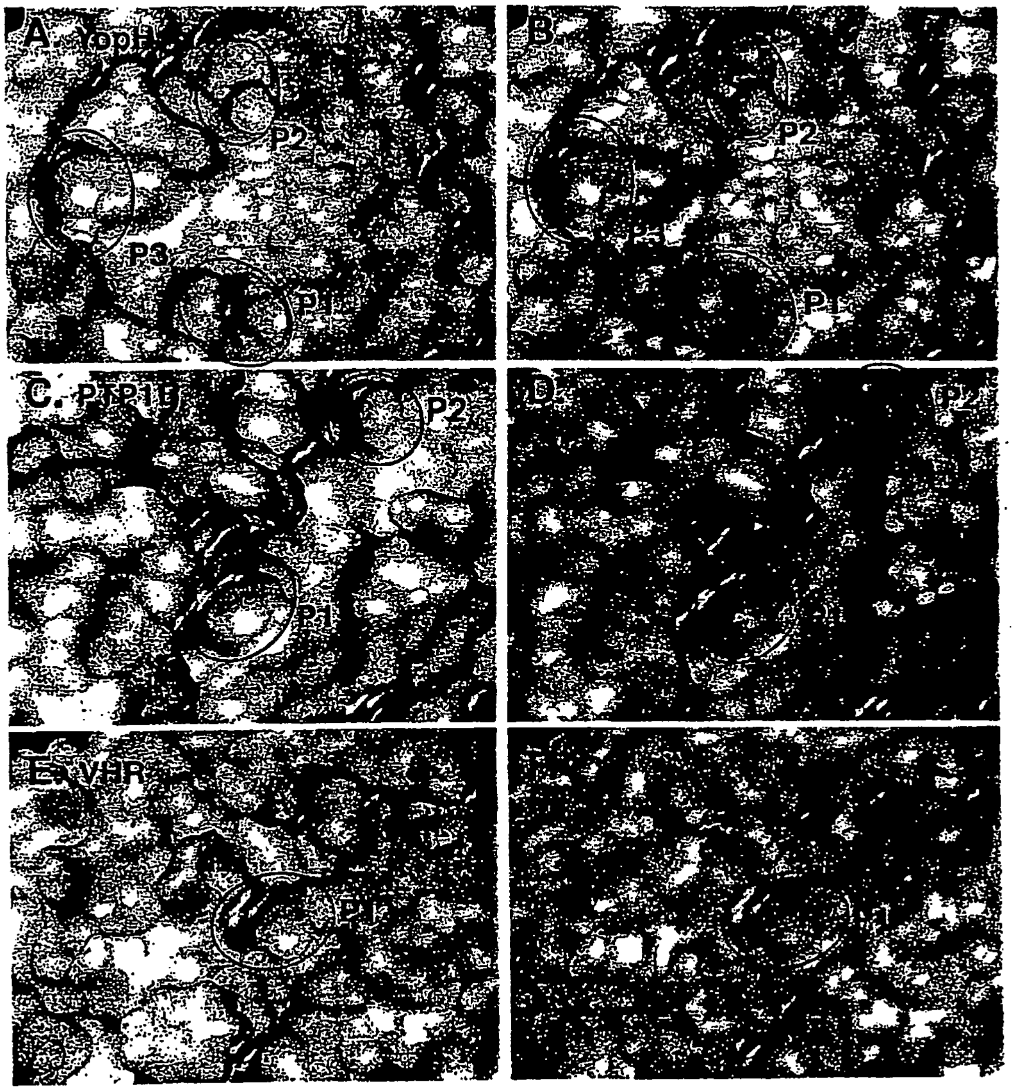


Figure 3

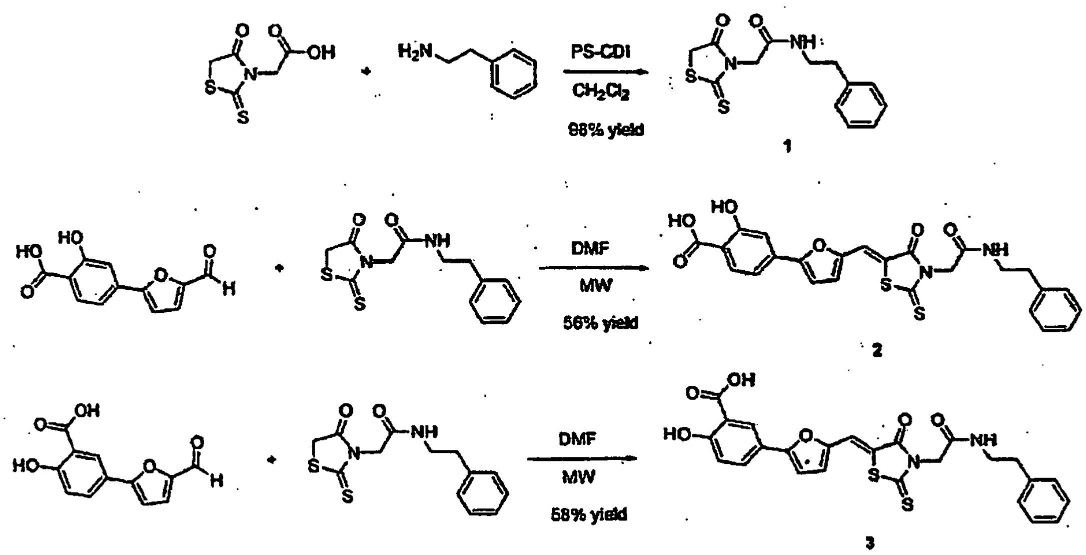


**Figure 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03097621 A


### Non-patent literature cited in the description


- **Ernst.** *Cell. Microbiol.,* 2000, vol. 2, 379-386
- **DeVinney et al.** *Trends Microbiol.,* 2000, vol. 8, 29-33
- **Persson et al.** *Mol. Microbiol.,* 1995, vol. 18, 135-150
- **Cheng et al.** *J. Bacteriol.,* 2000, vol. 182, 3183-3190
- **Autenrieth et al.** *Infect. Immun.,* 1993, vol. 61, 2585-2595
- **Autenrieth et al.** *Immunobiology,* 1993, vol. 187, 1-16
- **Autenrieth et al.** *J. Med. Microbiol.,* 1996, vol. 44, 285-294
- **Titball et al.** *British Medical Bulletin,* 1998, vol. 54, 625-633
- **Hinnebusch.** *J. Mol. Med.,* 1997, vol. 75, 645-652
- **Cleri et al.** *Semin. Resp. Infect.,* 1997, vol. 12, 12-23
- **Titball et al.** *Vaccine,* 2001, vol. 19, 4175-4184
- **Friedlander et al.** *Clin. Infect. Dis.,* 1995, vol. 21, 178-181
- **Christie.** *Ecol. Dis.,* 1982, vol. 1, 111-115
- **McGovern et al.** *Arch. Dermatol.,* 1999, vol. 135, 311-322
- **Inglesby et al.** *JAMA,* 2000, vol. 283, 2281-2290
- **Hawley et al.** *Annu. Rev. Microbiol.,* 2001, vol. 55, 235-253
- **Galimand et al.** *N. Engl. J. Med.,* 1997, vol. 337, 677-680
- **Guiyoule et al.** *Emerg. Infect. Dis.,* 2001, vol. 7, 43-48
- **Cornelis et al.** *Mol. Microbiol.,* 1997, vol. 23, 861-867
- **Cornelis et al.** *Microbiol. Mol. Biol. Rev.,* 1998, vol. 62, 1315-1352
- **Juris et al.** *Cellular Microbiology,* 2002, vol. 4, 201-211
- **Black et al.** *EMBO J.,* 1997, vol. 16, 2730-2744
- **Persson et al.** *EMBO J.,* 1997, vol. 16, 2307-2318
- **Andersson et al.** *Mol. Microbiol.,* 1996, vol. 20, 1057-1069
- **Aepfelbacher et al.** *Biol. Chem.,* 1999, vol. 380, 795-802
- **Green et al.** *J. Leuk Biol.,* 1995, vol. 57, 972-977
- **Yao et al.** *J. Exp. Med.,* vol. 190, 1343-1350
- **Cornelis.** *Proc. Natl. Acad. Sci. USA,* 2000, vol. 97, 8778-8783
- **Guan et al.** *Science,* 1990, vol. 249, 553-556
- **Bliska et al.** *Proc. Natl. Acad. Sci USA,* 1991, vol. 88, 1187-1191
- **Montagna et al.** *J. Biol. Chem.,* 2001, vol. 276, 5005-5011
- **Evdokimov et al.** *Acta Cryst.,* 2001, vol. 57, 793-799
- **Stuckey et al.** *Nature,* 1994, vol. 370, 571-575
- **Bliska et al.** *Proc. Natl. Acad Sci USA,* 1991, vol. 88, 1187-1191
- **Bliska et al.** *J. Exp. Med.,* 1992, vol. 176, 1625-1630
- **Hartland et al.** *Infect. Immun.,* 1994, vol. 62, 4445-4453
- **Black et al.** *Mol. Microbiol.,* 1998, vol. 29, 1263-1274
- **Ruckdeschet et al.** *Inject. Immun.,* 1996, vol. 84, 724-733
- **Liang et al.** *J. Biol. Chem.,* 2003, vol. 278, 41734-41741
- **Doman et al.** *J. Med. Chem.,* 2002, vol. 45, 2213-2221
- **Chen Yen Ting et al.** *Bio. Med. Chem.,* 2004, vol. 12, 3289-3298
- **Montagna et al.** *J. Biol. Chem.,* 2001, vol. 276, 5 005-5011
- **Ruckdeschel et al.** *Infect. Immun.,* 1996, vol. 64, 724-733
- **Yao et al.** *J. Exp. Med.,* 1999, vol. 190, 1343-1350
- **Alonso et al.** *J. Biol. Chem.,* 2004, vol. 279, 4922-4928

- **Sauvonnet et al.** *Mol. Microbiol.,* 2002, vol. 45, 805-815
- **Bliska et al.** *Proc. Natl. Acad. Sci US4,* 1991, vol. 88, 1187-1191
- **Bliska.** *J. Exp. Med.,* 1992, vol. 176, 1625-1630
- **Bolin et al.** *Mol. Microbiol.,* 1988, vol. 2, 237-245
- **Michielis et al.** *Microbial. Pathogen,* 1988, vol. 5, 449-459
- **Rosqvist et al.** *Infect. Immun.,* 1988, vol. 56, 2139-2143
- **Straley et al.** *Infect. Immun.,* 1986, vol. 51, 445-454
- **Salmeen et al.** *Mol. Cell.,* 2000, vol. 6, 1401-1412
- **Crowther, John R.** ELISA Theory and Practice. Humana Press, 1995
- Remington's Phannaceutical Sciences. Mack Publishing Co, 1990
- **Fingl et al.** The Pharmacological Basis of Therapeutics. 1975, 1
- **YopH. Stuckey et al.** *Nature,* 1994, vol. 370, 571-575
- **Barford et al.** *Science,* 1994, vol. 263, 1397-1404
- **Yuvaniyama et al.** *Science,* 1996, vol. 272, 1328-1331
- **Alonso et al.** *J Biol Chem,* 2002, vol. 277, 5524-5528
- **Alonso et al.** *Nat Immunol,* 2003, vol. 4, 44-48
- **Kholod.** *Biotechniques,* 2001, vol. 31, 322-328
- **Saxena et al.** *Nature Cell Biol.,* 1999, vol. 1, 305-311
- **Saxena et al.** *J. Biol. Chem.,* 1998, vol. 273, 15340-15344